# EUROPEAN PATENT APPLICATION

(11) **EP 3 846 460 A1**
(43) Date of publication of application: **07.07.2021**
(21) Application number: 19873856.9
(22) Date of filing: 09.10.2019
(51) Int. Cl.: H04N 7/18, A61B 1/00, A61B 1/045, A61B 1/06, H04N 13/221, H04N 13/239, H04N 13/254, H04N 13/271, H04N 13/296

(54) **MEDICAL-USE OBSERVATION SYSTEM, MEDICAL-USE OBSERVATION DEVICE AND MEDICAL-USE OBSERVATION METHOD**

(30) Priority: 18.10.2018 JP 2018196648
(71) Applicant: Sony Corporation, 108-0075 Tokyo (JP)
(72) Inventor: UYAMA, Keisuke, Tokyo 108-0075 (JP); HAYASHI, Tsuneo, Tokyo 108-0075 (JP)
(74) Representative: D Young & Co LLP
(86) International application number: PCT/JP2019/039738
(87) International publication number: WO 2020/080209

(57) **Abstract**

A three-dimensional information generation unit (14) generates a three-dimensional map (D(X, Y, Z)) (three-dimensional information) of a surgical field from a surgical field image (K(x, y)) that is captured by an imaging apparatus (42a). Then, a region-of-interest setting unit (20) (setting unit) sets at least one region of interest in a surgical field image that is captured at a predetermined timing. A detection region estimation unit (22) (estimation unit) estimates a relative position corresponding to the region of interest in a surgical field image that is captured at a certain timing different from the predetermined timing, on the basis of the three-dimensional map information on the region of interest. Then, a parameter control unit (26) (adjustment unit) adjusts imaging parameters of the imaging apparatus and a light source apparatus (60) that is a peripheral device of the imaging apparatus on the basis of three-dimensional information and pixel values of a surgical field image corresponding to the relative position of the region of interest estimated by the estimation unit, captures a surgical field image, and a display control unit (40) outputs the captured surgical field image.

## Description

### Field

The present disclosure relates to a medical observation system, a medical observation apparatus, and a medical observation method.

### Background

In recent years, surgery using a surgical endoscope or a surgical microscope that is provided with an auto focus (AF) function and an auto exposure (AE) function to implement a function to automatically adjust imaging parameters of an image is performed. The endoscope and the microscope generally have a shallow depth of field, and a surgical field usually has clear contrast. Therefore, when an image of a subject is to be captured, it is desirable to continuously adjust imaging parameters, such as focus and exposure, in accordance with a site (surgical site) to be subjected to surgery. For example, in Patent Literature 1, an endoscope system that divides an endoscope image into a plurality of divided regions and selects a region to be subjected to focus control is proposed.

### Citation List

### Patent Literature

Patent Literature 1: JP 2011-139760 A

### Summary

### Technical Problem

However, an object, such as a surgical instrument or a hand of a surgeon, other than the surgical site often appears in a surgical field image, and in some cases, the surgical site may overlap with the surgical instrument, so that it is difficult to continuously adjust the imaging parameters with accuracy in accordance with the surgical site by using only two-dimensional information, such as the surgical field image.

In view of the above, the present disclosure proposes a medical observation system, a medical observation apparatus, and a medical observation method capable of continuously adjusting focus and exposure with accuracy in accordance with a surgical field.

### Solution to Problem

In order to solve the problem described above, a medical observation system is provided that includes: an imaging apparatus that obtains a surgical field image by capturing an image of a surgical field; a three-dimensional information generation unit that generates three-dimensional information on a surgical field from the surgical field image captured by the imaging apparatus; a setting unit that sets at least one region of interest in a surgical field image that is captured by the imaging apparatus at a predetermined timing; an estimation unit that estimates a relative position corresponding to the region of interest from a surgical field image that is captured at a certain timing different from the predetermined timing, on the basis of the three-dimensional information and information on the region of interest; an adjustment unit that adjusts a control parameter of the imaging apparatus when the imaging apparatus captures a surgical field image, on the basis of three-dimensional information and pixel values of a surgical field image corresponding to the relative position of the region of interest estimated by the estimation unit; and a display control unit that outputs a surgical field image that is captured by the imaging apparatus using the control parameter adjusted by the adjustment unit.

### Brief Description of Drawings

FIG. 1 is a diagram illustrating an example of a schematic configuration of an endoscope surgical system to which a medical observation system according to a first embodiment of the present disclosure is applicable.
FIG. 2 is a diagram illustrating an example of a schematic configuration of the medical observation system according to the first embodiment of the present disclosure.
FIG. 3 is a diagram for explaining a method of generating a three-dimensional map of a surgical field by a map generation unit.
FIG. 4 is a diagram illustrating an example of a method of setting a frame of interest.
FIG. 5 is a diagram illustrating another example of the method of setting the frame of interest.
FIG. 6 is a diagram illustrating an example of setting of a region for extracting feature points.
FIG. 7 is a diagram illustrating an example of a surgical field image that is displayed by the medical observation system.
FIG. 8 is a diagram for explaining a function of the medical observation system to change an imaging magnification at the time of observation in accordance with a distance to a surgical field.
FIG. 9 is a flowchart illustrating an example of the flow of a process performed by the medical observation system.
FIG. 10 is a diagram illustrating an example of a schematic configuration of a medical observation system in which an imaging apparatus includes an imaging element with an imaging plane phase difference sensor.
FIG. 11 is a diagram illustrating an example of a display mode of a surgical field image when the imaging plane phase difference sensor is used.
FIG. 12 is a diagram illustrating an example of a schematic configuration of a medical observation system in which an imaging apparatus includes two imaging elements.
FIG. 13 is a diagram illustrating an example of a schematic configuration of a medical observation system in which an imaging apparatus includes two imaging elements and a camera control unit includes a tracking processing unit.
FIG. 14 is a diagram illustrating an example of a schematic configuration of a medical observation system in which an imaging apparatus includes an imaging element and a depth sensor.
FIG. 15 is a diagram illustrating an example of a schematic configuration of a medical observation system in which an imaging apparatus includes an imaging element and a depth sensor and a camera control unit includes a tracking processing unit.
FIG. 16 is a diagram illustrating an example of setting of a plurality of regions of interest in a surgical field image.
FIG. 17 is a diagram illustrating an example of a display mode in which a region in a predetermined distance range is displayed in a highlighted manner in a surgical field image.
FIG. 18 is a diagram illustrating examples of display modes of a frame of interest that is set in a surgical field image.
FIG. 19 is a diagram for explaining a function to detect a portion with intensive reflected light in a surgical field image.
FIG. 20 is a diagram illustrating an example of a schematic configuration of a microscope surgical system to which the technology according to the present disclosure is applicable.
FIG. 21 is a diagram illustrating a situation in which surgery is performed using the microscope surgical system.

### Description of Embodiments

Embodiments of the present disclosure will be described in detail below based on the drawings. In each of the embodiments below, the same components are denoted by the same reference symbols, and repeated explanation will be omitted.

### First Embodiment

### [Configuration of medical observation system according to first embodiment]

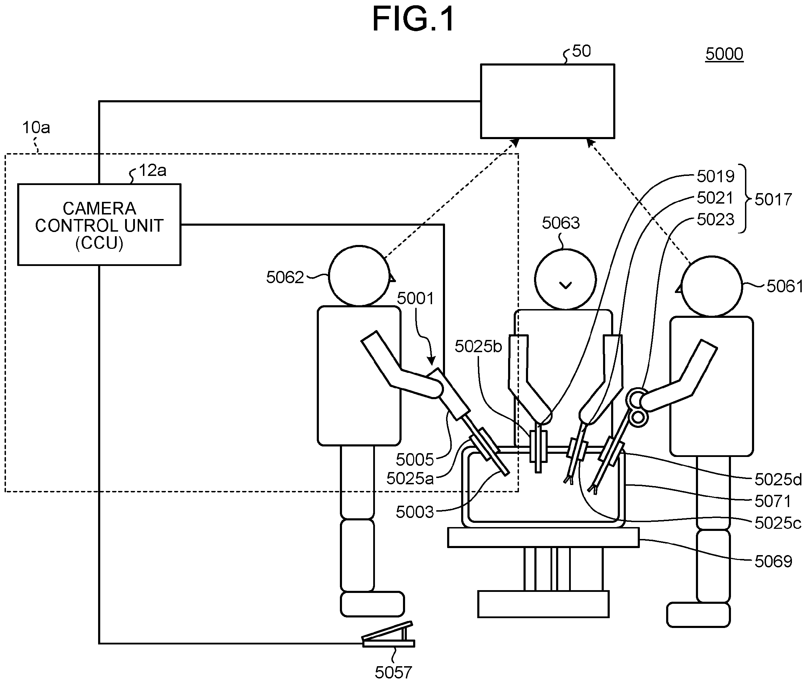

FIG. 1 is a diagram illustrating an example of a schematic configuration of an endoscope surgical system 5000 to which a medical observation system according to the present disclosure is applicable. FIG. 1 illustrates a state in which a surgeon (a doctor or an operator) 5061 performs surgery on a patient 5071 on a patient bed 5069 by using the endoscope surgical system 5000. A scopist 5062 holds an endoscope 5001 and inserts the endoscope 5001 into a body cavity of the patient 5071. An assistant 5063 holds surgical tools 5017 and inserts the surgical tools 5017 into the body cavity of the patient 5071.

In endoscopy surgery, an abdominal wall is punctured at a plurality of positions by a plurality of cylindrical drilling tools called trocars 5025a to 5025d, instead of cutting and opening the abdominal wall. Then, a lens barrel 5003 of the endoscope 5001 and the other surgery tools 5017 are inserted into the body cavity of the patient 5071 through the trocars 5025a to 5025d. In the example in FIG. 1, an insufflation tube 5019, an energy treatment tool 5021, and a forceps 5023 are inserted as the other surgery tools 5017 into the body cavity of the patient 5071. The insufflation tube 5019 transmits gas into the body cavity to inflate the body cavity in order to ensure a visual field of the endoscope 5001 and ensure an operation space of the surgeon 5061. The energy treatment tool 5021 is a treatment tool for making an incision in and separate tissue, sealing a blood vessel, and the like using a high-frequency current or ultrasonic vibration. Further, although not illustrated in FIG. 1, the insufflation tube 5019 and the energy treatment tool 5021 are connected to a control device (not illustrated), and the surgical tools 5017 that have received instructions from the surgeon 5061 or the like perform predetermined operation. Meanwhile, the surgical tools 5017 illustrated in the drawing are mere examples, and various surgical tools, such as tweezers and a retractor, which are typically used in endoscopic surgery may be used as the surgical tools 5017.

An image (hereinafter, referred to as a surgical field image) of a surgical field inside the body cavity of the patient 5071 captured by the endoscope 5001 is displayed on a display apparatus 50. The surgeon 5061 performs treatment, such as excision of an affected area, by using the energy treatment tool 5021 and the forceps 5023 while watching the surgical field image displayed on the display apparatus 50 in real time. Further, the scopist 5062 adjusts a position of the endoscope 5001 such that the affected area appears in the surgical field image while viewing the surgical field image displayed on the display apparatus 50 in real time. Meanwhile, the insufflation tube 5019, the energy treatment tool 5021, and the forceps 5023 are held by the surgeon 5061, the assistant 5063, and the like during surgery.

### [Schematic configuration of endoscope]

The endoscope 5001 includes the lens barrel 5003, in which a region with a certain length from a distal end is inserted into the body cavity of the patient 5071, and a camera head 5005 that is connected to a proximal end of the lens barrel 5003. In the example in FIG. 1, the endoscope 5001 that is configured as what is called a rigid scope including the rigid lens barrel 5003 is illustrated, but the endoscope 5001 may be configured as what is called a flexible scope including the flexible lens barrel 5003.

An opening in which an objective lens is fitted is arranged at the distal end of the lens barrel 5003. A light source apparatus (not illustrated) is connected to the endoscope 5001, and light generated by the light source apparatus is guided to the distal end of the lens barrel 5003 by a light guide that is extended inside the lens barrel 5003, and applied to an observation target inside the body cavity of the patient 5071 via the objective lens. Meanwhile, the endoscope 5001 may be a forward-viewing endoscope, a forward-oblique viewing endoscope, or a side-viewing endoscope.

An optical system and an imaging element are arranged inside the camera head 5005, and reflected light (observation light) from the observation target is collected on the imaging element by the optical system. The imaging element performs photoelectric conversion on the observation light, and generates an electrical signal corresponding to the observation light, that is, an image signal corresponding to an observation image. The image signal is transmitted, as RAW data, to a camera control unit (CCU) 12a. Meanwhile, the camera head 5005 has a function to adjust an imaging magnification and a focal length by appropriately driving the optical system.

Furthermore, for example, the camera head 5005 may include a plurality of imaging elements in order to cope with a stereoscopic view (3D-display) or the like. In this case, a plurality of relay optical systems are arranged inside the lens barrel 5003 in order to guide the observation light to the respective imaging elements.

The endoscope surgical system 5000 includes an input device that receives input of various kinds of information and input of instructions from the surgeon 5061, the scopist 5062, or the assistant 5063 who is a user. For example, the user inputs, via the input device, various kinds of information on surgery, such as body information on a patient and information on a surgery procedure. Furthermore, for example, the user inputs, via the input device, an instruction to change imaging conditions (a type of illumination light, the imaging magnification, the focal length, and the like) for the endoscope 5001, an instruction to drive the surgical tools 5017, such as the energy treatment tool 5021, and the like.

A type of the input device is not specifically limited, and various known input devices may be adopted as the input device. As the input device, for example, a mouse, a keyboard, a touch panel, a switch and/or a lever may be adopted. FIG. 1 illustrates an example in which the scopist 5062 inputs information using a foot switch 5057 that is one example of the input device. For example, the scopist 5062 sets a region of interest in the surgical field image via the foot switch 5057. This will be described in detail later. Meanwhile, if a touch panel is used as the input device, the touch panel may be arranged on a display screen of the display apparatus 50.

### Description of configuration of medical observation system according to first embodiment

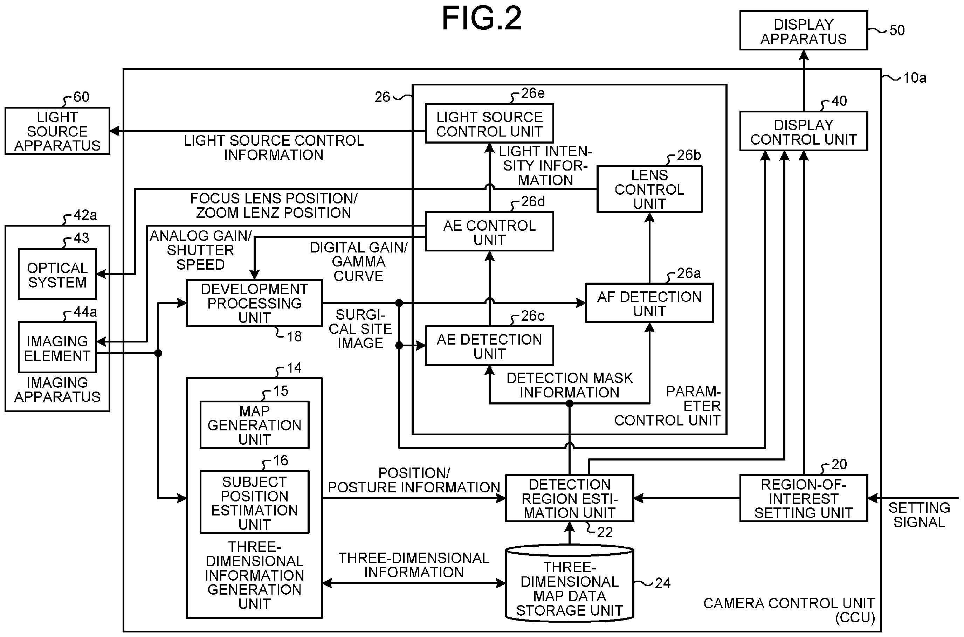

FIG. 2 is a functional block diagram illustrating a functional configuration of a medical observation system 10a that is applied to endoscopy surgery. The medical observation system 10a is a system that is applied to, for example, the endoscope surgical system 5000 as described above, and monitors a surgical field image using the endoscope 5001 that is inserted into the body cavity of the patient 5071 during surgery. In particular, the medical observation system 10a is a system that displays an enlarged surgical field image in which a set region of interest is always enlarged, independent of a positon and posture of the endoscope 5001, on the basis of a three-dimensional position of the surgical field.

The medical observation system 10a includes an imaging apparatus 42a and a camera control unit 12a. The imaging apparatus 42a is mounted on the camera head 5005 of the endoscope 5001 as described above, captures an image of a surgical field inside the body cavity of the patient 5071, and obtains a surgical field image. When the imaging apparatus 42a performs imaging, the camera control unit 12a generates the surgical field image and also generates three-dimensional information on the surgical field.

The imaging apparatus 42a includes an optical system 43 and an imaging element 44a. The optical system 43 is, for example, a lens that has an automatic focus function and an angle-of-view adjustment function (zoom function). The imaging element 44a is configured with an imaging element (photoelectric conversion element), such as a complementary metal oxide semiconductor (CMOS) image sensor or a charge coupled device (CCD) image sensor, and coverts light received from the surgical field into an electrical signal.

The camera control unit 12a includes a three-dimensional information generation unit 14, a development processing unit 18, a region-of-interest setting unit 20, a detection region estimation unit 22, a three-dimensional map data storage unit 24, a parameter control unit 26, and a display control unit 40. The camera control unit 12a generates an enlarged surgical field image, in which the region of interest is always enlarged, independent of the position and the posture of the endoscope, and displays the enlarged surgical field image on the display apparatus 50. Meanwhile, the camera control unit 12a is one example of the medical observation apparatus according to the present disclosure.

The three-dimensional information generation unit 14 calculates a three-dimensional position of the surgical field image of the inside of the body cavity captured by the imaging element 44a, for example. The three-dimensional information generation unit 14 includes a map generation unit 15 and a subject position estimation unit 16. The map generation unit 15 generates a three-dimensional map (hereinafter, simply referred to as a map) that indicates the three-dimensional position of the surgical field and a three-dimensional position of the region of interest to be described later. A method of generating the map will be described later. The subject position estimation unit 16 estimates a subject position and posture of the endoscope 5001 at a predetermined timing, on the basis of the generated map and a surgical field image that is captured at the predetermined timing.

The development processing unit 18 converts captured data into a viewable image. The development processing unit 18 performs various kinds of image processing for displaying an image, such as a developing process (demosaicing process), on the RAW data that is output by the imaging element 44a. More specifically, the development processing unit 18 applies a digital gain or a gamma curve designated by an AE control unit 26d (to be described later) to the RAW data, and converts the RAW data to visible image data.

The region-of-interest setting unit 20 sets a region to which attention is paid, such as a tumor to be removed by surgery, in the surgical field image that is captured by the imaging element 44a and that is converted to a viewable image by the development processing unit 18. More specifically, an operator of the medical observation system 10a sets at least one region of interest in the surgical field image while monitoring the surgical field image on the display apparatus 50, such as a liquid crystal monitor. A detailed method of setting the region of interest will be described later. Meanwhile, the region-of-interest setting unit 20 is one example of a setting unit according to the present disclosure.

The detection region estimation unit 22 estimates, as a detection region, a relative position that corresponds to the region of interest in a surgical field image obtained at an arbitrary time. Here, the relative position is a position corresponding to a physical position of a region of interest that is set in a frame obtained at a predetermined timing, and is, for example, an object region corresponding to the physical position in a frame obtained at a different timing. Meanwhile, the detection region estimation unit 22 is one example of an estimation unit according to the present disclosure.

The three-dimensional map data storage unit 24 stores therein the three-dimensional map of the surgical field generated by the map generation unit 15 as described above. Meanwhile, the three-dimensional map stored in the three-dimensional map data storage unit 24 is updated with time.

The parameter control unit 26 adjusts control parameters of the imaging apparatus 42a and a light source apparatus 60, on the basis of three-dimensional information and pixel values of the surgical field image at the relative position that corresponds to the region of interest and that is estimated by the detection region estimation unit 22. Meanwhile, the parameter control unit 26 is one example of an adjustment unit according to the present disclosure. Here, the control parameters that are adjusted by the parameter control unit 26 include an optical parameter, an imaging parameter, a development parameter, and a light emission parameter.

The optical parameter is a parameter for defining a state of the optical system of the imaging apparatus 42a. Specifically, the optical parameter includes a focus position, an angle of view, an aperture value, and the like of the optical system 43.

The imaging parameter is a parameter for defining an exposure condition of the imaging apparatus 42a. Specifically, the imaging parameter includes a shutter speed, a gain value, and the like for exposing the imaging element 44a.

The development parameter is a parameter for defining a development condition of the imaging apparatus 42a. Specifically, the development parameter includes a digital gain, a gamma curve, and the like that are the development conditions of the development processing unit 18.

The light emission parameter is a parameter for defining a light emission state (a light emission amount and a light emission time) of the light source apparatus 60 that applies illumination light to an imaging range of the imaging apparatus 42a. Meanwhile, the light source apparatus 60 is, for example, a light emitting diode (LED). Further, the light emission parameter includes, in particular, a wavelength, light intensity, a light emission timing, and the like.

The parameter control unit 26 implements the AF function and the AE function of the imaging apparatus 42a by controlling the parameters as described above. The AF function is a function to adjust a focus position of a surgical field image K(x, y) that is captured by the imaging apparatus 42a. The AE function is a function to adjust exposure of the surgical field image K(x, y) that is captured by the imaging apparatus 42a.

More specifically, the parameter control unit 26 includes an AF detection unit 26a, a lens control unit 26b, an AE detection unit 26c, the AE control unit 26d, and a light source control unit 26e.

The AF detection unit 26a extracts information (detection value) indicating an in-focus state with respect to the region of interest, from a distribution of pixel values in a region at the relative position that corresponds to the region of interest and that is estimated by the detection region estimation unit 22 in the surgical field image K(x, y) that is captured by the imaging apparatus 42a. Examples of the information indicating the in-focus state includes information representing a difference between lightness and darkness, that is, contrast, in a frame of interest 110, an evaluation value that is obtained by evaluating the contrast in accordance with a predetermined criterion, and phase information if the imaging element includes a pixel with a phase difference.

The lens control unit 26b generates control data for controlling a focus lens position, a lens movement amount, and the like such that the position of the frame of interest 110 is focused, on the basis of the detection value extracted by the AF detection unit 26a. Further, the lens control unit 26b generates control data for controlling an angle of view of a lens included in the imaging apparatus 42a, on the basis of a distance to a region in which the frame of interest 110 is set. Then, the lens control unit 26b transmits the generated control data to the optical system 43.

The AE detection unit 26c extracts pixel information (detection value) that is needed to adjust exposure, from a distribution (histogram) of pixel values in a region corresponding to an estimated position of the region of interest that is estimated by the detection region estimation unit 22 in the surgical field image K(x, y) that is captured by the imaging apparatus 42a. The pixel information that is needed to adjust exposure of the region of interest is, for example, a statistic, such as a difference between a maximum value and a minimum value of the pixel values (difference between lightness and darkness), an average value of the pixel values, or a variance of the pixel values, that is calculated from the distribution of the pixel values in the frame of interest 110.

The AE control unit 26d calculates control parameters including a digital gain and a gamma curve such that the surgical field image K(x, y) that can be viewed easily by the surgeon 5061 is captured, on the basis of the detection value that is extracted by the AE detection unit 26c. Then, the AE control unit 26d transmits the calculated control parameters including the digital gain and the gamma curve to the development processing unit 18.

The digital gain is a gain that is used to perform amplification after the RAW data output by the imaging element 44a is converted to a digital signal by an AD converter (not illustrated) included in the development processing unit 18. The digital gain is used when a developed image is subjected to post-processing, and is used for limb darkening correction, white balance adjustment, exposure adjustment, and the like.

The gamma curve indicates a correction characteristic for correcting the pixel values such that the surgical field image K(x, y) can be displayed with appropriate brightness when displayed on the display apparatus 50. In general, the pixel values of the image displayed on the display apparatus 50 and brightness of a screen do not have a proportional relationship. Therefore, if the pixel values are displayed as they are on the display apparatus 50, in some cases, brightness balance may be disturbed. Therefore, in general, pre-processing is performed by performing gamma correction on the pixel values such that the pixel values subjected to the gamma correction and the brightness of the screen have a proportional relationship. The gamma curve indicates the correction characteristic that is adopted when the gamma correction is performed.

The AE control unit 26d further calculates control parameters including an analog gain and a shutter speed such that the surgical field image K(x, y) that can be viewed easily by the surgeon 5061 is captured, on the basis of the detection value that is extracted by the AE detection unit 26c. Then, the AE control unit 26d transmits the calculated control parameters including the analog gain and the shutter speed to the optical system 43. Further, the AE control unit 26d calculates light intensity information for controlling the light source apparatus 60 such that the surgical field image K(x, y) that can be viewed easily by the surgeon 5061 is captured, on the basis of the information that is calculated by the AE detection unit 26c. Then, the AE control unit 26d transmits the calculated light intensity information to the light source control unit 26e.

The analog gain is a gain that is used to amplify the RAW data that is an analog signal output by the imaging element 44a. The analog gain is used before the developing process is performed, and is used to adjust ISO sensitivity.

Further, the AE control unit 26d calculates light intensity information that is used to cause the light source apparatus 60 to emit light such that the surgical field image K(x, y) that can be viewed easily by the surgeon 5061 is captured, on the basis of the detection value that is extracted by the AE detection unit 26c. Then, the AE control unit 26d transmits control parameters including the calculated light intensity information to the light source control unit 26e.

The light intensity information is information indicating light intensity at which the light source apparatus 60 emits light, for example.

Meanwhile, the AE control unit 26d may transmit parameters including not only the exposure adjustment of the region of interest, but also white balance, color correction, and the gamma curve to the development processing unit 18 so that tissue in the region of interest can easily be observed.

The light source control unit 26e generates light source control information that is a driving signal for causing the light source apparatus 60 to actually emit light, on the basis of the light intensity information that is calculated by the AE control unit 26d. Then, the light source control unit 26e transmits the generated light source control information to the light source apparatus 60. Meanwhile, the light source control unit 26e may improve visibility of tissue in the surgical field image K(x, y) by controlling, as the control parameter, emission color (emission wavelength) of the light source apparatus 60 on the basis of the detection value that is detected by the AE detection unit 26c.

The imaging apparatus 42a performs imaging using the imaging parameter that is adjusted by the parameter control unit 26, at a timing at which the light source apparatus 60 that is driven with the light source control information adjusted by the parameter control unit 26 performs illumination. Then, the development processing unit 18 performs the developing process on the image that is captured by the imaging apparatus 42a, by using the development parameter that is adjusted by the parameter control unit 26. Then, the display control unit 40 performs display control of outputting the image subjected to the developing process by the development processing unit 18 to the display apparatus 50. Meanwhile, as the display apparatus 50, various known display apparatuses, such as a liquid crystal display apparatus or an electro luminescence (EL) display apparatus may be adopted.

Meanwhile, the parameter control unit 26 may be configured to perform all of the AF control, the AE control, and the light source control as described above, or perform only a part of the above-described control.

### [Description of method of generating three-dimensional map]

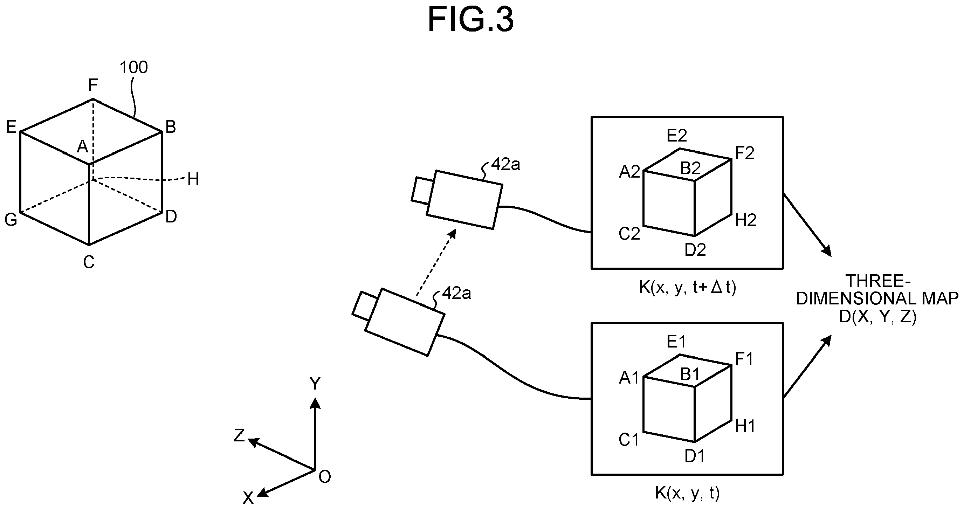

A method of generating a three-dimensional map of a surgical field by the map generation unit 15 will be described below. FIG. 3 is a diagram for explaining the method of generating the three-dimensional map of the surgical field by the map generation unit 15.

FIG. 3 illustrates a state in which an object 100 that stands still is observed by the imaging apparatus 42a in a three-dimensional space XYZ in which a spatial point is adopted as a reference position O. Further, it is assumed that the imaging apparatus 42a captures a surgical field image K(x, y, t) at a time t, and captures a surgical field image K(x, y, t+Δt) at a time t+Δt. Meanwhile, a time interval Δt is set to a predetermined time, such as 33 milliseconds (msec). Furthermore, the reference position O may be set arbitrarily, but is preferably set to a position that is not moved with time, for example.

The map generation unit 15 first detects a feature point that is a characteristic point (pixel) from each of the surgical field image K(x, y, t) and the surgical field image K(x, y, t+Δt). The feature point is, for example, a pixel with a pixel value that is different by a predetermined value or larger as compared to neighboring pixels. Meanwhile, it is preferable that the feature point is a point that is stably present over time, and, for example, a pixel constituting an edge in the image is often used. Here, for simplicity of explanation below, it is assumed that feature points A1, B1, C1, D1, E1, F1, and H1 that are vertices of the object are detected from the surgical field image K(x, y, t).

Subsequently, the map generation unit 15 searches for points corresponding to the feature points A1, B1, C1, D1, E1, F1, and H1 from the surgical field image K(x, y, t+Δt). Specifically, points with similar features are searched for from the surgical field image K(x, y, t+Δt) on the basis of a pixel value of the feature point A1, pixel values near the feature point A1, and the like. It is assumed that, through the search process as described above, feature points A2, B2, C2, D2, E2, F2, and H2 respectively corresponding to the feature points A1, B1, C1, D1, E1, F1, and H1 are detected from the surgical field image K(x, y, t+Δt) .

Subsequently, the map generation unit 15 calculates a three-dimensional coordinate (X_{A}, Y_{A}, Z_{A}) of a spatial point A from a two-dimensional coordinate of the feature point A1 in the surgical field image K(x, y, t+Δt) and a two-dimensional coordinate of the feature point A2 in the surgical field image K(x, y, t+Δt) on the basis of the principle of three-dimensional measurement, for example. As a set of the three-dimensional coordinates (X_{A}, Y_{A}, Z_{A}) calculated as described above, a three-dimensional map D(X, Y, Z) of the space in which the object 100 is placed is generated. The generated three-dimensional map D(X, Y, Z) is stored in the three-dimensional map data storage unit 24. Meanwhile, the three-dimensional map D(X, Y, Z) is one example of three-dimensional information according to the present disclosure.

Meanwhile, a position and posture of the imaging apparatus 42a are changed during the time interval Δt, so that the map generation unit 15 simultaneously estimates the position and the posture of the imaging apparatus 42a. Mathematically, simultaneous equations based on the two-dimensional coordinates of the feature points observed in the surgical field image K(x, y, t) and the surgical field image K(x, y, t+Δt) are set up while the three-dimensional coordinate of each of the feature points of the object 100 and the position of the imaging apparatus 42a are adopted as unknowns. By solving the simultaneous equations as described above, the map generation unit 15 estimates the three-dimensional coordinate of each of the feature points of the object 100 and the position and the posture of the imaging apparatus 42a.

In this manner, by detecting a plurality of feature points from the surgical field image K(x, y, t) that is captured by the imaging apparatus 42a, and detecting points corresponding to the feature points from the surgical field image K(x, y, t+Δt), it is possible to generate the three-dimensional map D(X, Y, Z) of an environment that is observed by the imaging apparatus 42a. Furthermore, it is possible to estimate the position and the posture, that is, the subject position, of the imaging apparatus 42a. Moreover, by repeatedly performing the process as described above, feature points that are not viewed at the beginning become visible, so that it is possible to improve the three-dimensional map D(X, Y, Z). Moreover, by repeating the process, it is possible to repeatedly calculate the three-dimensional position of the same feature point, so that it is possible to reduce a calculation error by performing an averaging process, for example. In this manner, the three-dimensional map D(X, Y, Z) stored in the three-dimensional map data storage unit 24 is updated as needed. Meanwhile, a technique of generating the three-dimensional map of the environment and identifying the subject position of the imaging apparatus 42a is generally called a simultaneous localization and mapping (SLAM) technique.

The basic principle of the SLAM technique using a monocular camera is described in, for example, "Andrew J. Davison, "Real-Time Simultaneous Localization and Mapping with a Single Camera", Proceedings of the 9th IEEE International Conference on Computer Vision Volume 2, 2003, pp. 1403-1410". Further, the SLAM technique for estimating the three-dimensional position of an object using a camera image of the object is particularly referred to as Visual SLAM.

### [Description of method of setting region of interest]

The region of interest is set by operation of the region-of-interest setting unit 20. Specifically, the region-of-interest setting unit 20 displays a frame of interest indicating a region of interest on the surgical field image in a superimposed manner, and specifies a size, a shape, and a position of the frame of interest.

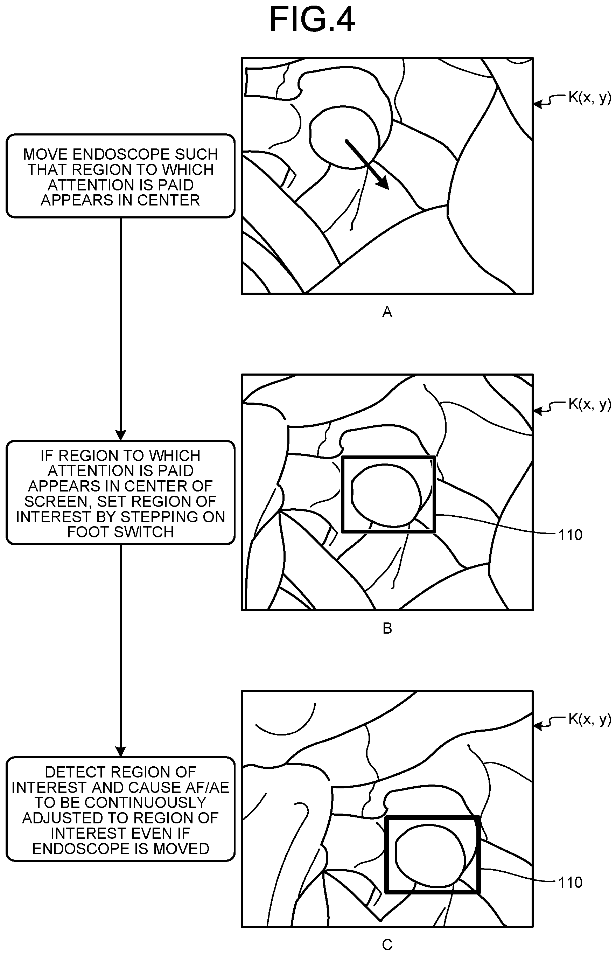

FIG. 4 is a diagram illustrating an example of a method of setting the frame of interest. FIG. 4A is a diagram illustrating an example of the surgical field image K(x, y) that is observed by the endoscope 5001. Meanwhile, in the description below, the surgical field image is simply denoted by K(x, y) by omitting information on a time at which the surgical field image is captured. FIG. 4B is a diagram illustrating an example of a state in which an orientation of the endoscope 5001 is adjusted such that an affected area that is to be set as a region of interest appears in the center of the surgical field image K(x, y) and the region-of-interest setting unit 20 sets the frame of interest 110 indicating the region of interest. FIG. 4C is a diagram illustrating an example of the surgical field image K(x, y) for which the camera control unit 12a performs control of continuously adjusting AF and AE with respect to the set region of interest.

The scopist 5062 moves the endoscope 5001 such that a specific position, such as an affected area, that is to be enlarged appears in the center (one example of a predetermined position) of the surgical field image K(x, y) while viewing the surgical field image K(x, y) illustrated in FIG. 4A, for example.

As illustrated in FIG. 4B, if the specific position to which attention is paid appears in the center (one example of the predetermined position) of the surgical field image K(x, y), the scopist 5062 steps on the foot switch 5057 (FIG. 1) and gives an instruction to set a region of interest to the region-of-interest setting unit 20. At this time, a setting signal for giving the instruction to set the region of interest is generated by using the operation of stepping on the foot switch 5057 as a trigger. Then, the region-of-interest setting unit 20 sets the region of interest by displaying the frame of interest 110 with a predetermined size in the center of the surgical field image K(x, y) as illustrated in FIG. 4B, under the condition that the setting signal is input. Meanwhile, the size and the shape of the frame of interest 110 may be set arbitrarily.

Subsequently, the parameter control unit 26 calculates control parameters of the imaging apparatus 42a, the camera control unit 12a, and the light source apparatus 60 on the basis of the detection value that is calculated inside a detection region, where the detection region is the inside of the frame of interest 110 that is set in FIG. 4B. Thereafter, the parameter control unit 26 controls the imaging apparatus 42a, the camera control unit 12a, and the light source apparatus 60 using the calculated control parameters and captures the surgical field image K(x, y). Then, the display control unit 40 outputs the captured surgical field image K(x, y) to the display apparatus 50 as illustrated in FIG. 4C. At this time, the position and the posture of the endoscope 5001 are changed, so that the position of the frame of interest 110 in the surgical field image K(x, y) is changed; however, the inside of the frame of interest 110 is continuously focused and displayed with brightness with which observation can be performed easily. The surgeon 5061 performs surgery while observing the surgical field image K(x, y) as illustrated in FIG. 4C.

Meanwhile, the method of setting the region of interest by the region-of-interest setting unit 20 is not limited by the method as described above. For example, it may be possible to set the position and the shape of the region of interest by mounting a touch panel in a laminated manner on the screen of the display apparatus 50 and detecting operation on the touch panel. Furthermore, it may be possible to set the position and the shape of the region of interest by using a mouse.

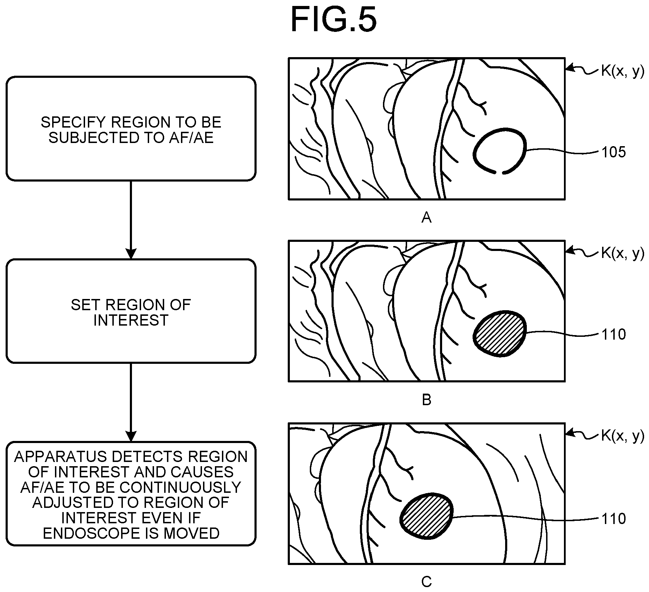

FIG. 5 is a diagram illustrating another example of the method of setting the frame of interest. FIG. 5A is a diagram illustrating an example of the surgical field image K(x, y) that is observed by the endoscope 5001. The scopist 5062 designates, by the input device, such as a touch panel or a mouse, a position of a region to which attention is paid, while viewing the surgical field image K(x, y) that is displayed on the display apparatus 50. The region-of-interest setting unit 20 outputs region-of-interest designation information 105 that indicates the designated region in a superimposed manner on the surgical field image K(x, y).

Subsequently, the region-of-interest setting unit 20 sets the frame of interest 110 at a position indicated by the input region-of-interest designation information 105. The region-of-interest setting unit 20 outputs the set frame of interest 110 in a superimposed manner on the surgical field image K(x, y) as illustrated in FIG. 5B. Meanwhile, the frame of interest 110 may be a frame with a certain size and a certain shape that are set in advance, or may be a closed region that represents the region-of-interest designation information 105.

Thereafter, the medical observation system 10a generates the surgical field image K(x, y) in which focus and exposure are continuously adjusted to the set frame of interest 110 as illustrated in FIG. 5C, independent of the position and the posture of the endoscope 5001.

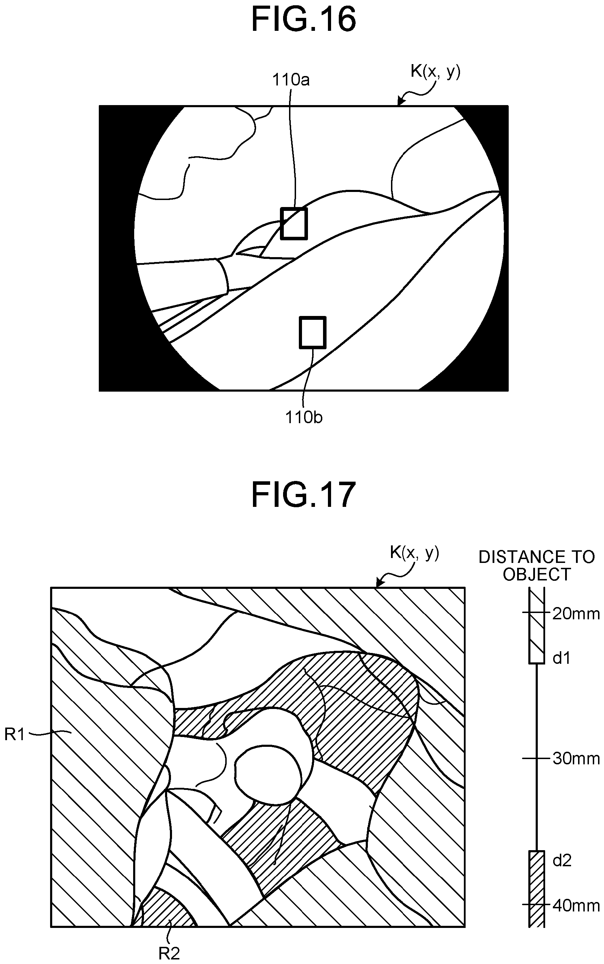

Meanwhile, the region-of-interest setting unit 20 may set the region of interest by additionally taking into account a condition that a distance in the three-dimensional space or a distance from the imaging system falls in a predetermined range by using the three-dimensional map D(X, Y, Z) as described above. Furthermore, the display mode of the frame of interest 110 is not limited to those illustrated in FIG. 4 and FIG. 5. Variations of the display mode of the frame of interest 110 will be described later (see FIG. 16). Moreover, the region-of-interest setting unit 20 may set the position and the shape of the region of interest on the basis of operation, such as a gesture.

### [Description of method of estimating relative position corresponding to region of interest

Thereafter, if the scopist 5062 moves the endoscope 5001, the detection region estimation unit 22 estimates a relative position corresponding to the region of interest in the surgical field image K(x, y). Then, the parameter control unit 26 performs the parameter adjustment as described above with respect to the estimated relative position corresponding to the region of interest. The display control unit 40 outputs the surgical field image K(x, y) that is captured using the adjusted parameters to the display apparatus 50. By continuing the process as described above, the medical observation system 10a causes the display apparatus 50 to continuously display the surgical field image K(x, y).

Here, a method of estimating the relative position that corresponds to the region of interest and that needs to be detected from the surgical field image K(x, y) by the detection region estimation unit 22 when the position or the posture of the endoscope 5001 is changed will be described.

The detection region estimation unit 22 estimates at which position in the surgical field image K(x, y, t+Δt) the frame of interest 110 that was observed at the time t is observed at the time t+Δt, that is, the relative position corresponding to the frame of interest 110, on the basis of, for example, the position and the posture of the endoscope 5001 at the time t, the position and the posture of the endoscope 5001 at a predetermined timing, e.g., at the time t+Δt that is different from the time t, and the three-dimensional map D(X, Y, Z).

Specifically, the detection region estimation unit 22 identifies how a plurality of feature points near the set frame of interest 110 have moved from the time t to the time t+Δt, on the basis of the position and the posture of the endoscope 5001. Then, the detection region estimation unit 22 estimates the relative position that corresponds to the frame of interest 110 and that needs to be detected, on the basis of information on the frame of interest 110, in particular, moving states of the identified feature points.

Meanwhile, in general, it is often the case that a region that is set as the region of interest is an affected area to be subjected to surgery. The affected area is highly likely to be largely deformed by being excised by surgery or by bleeding. Therefore, even if the feature points are set inside the region of interest, the feature points may disappear with time. To cope with this, it is preferable to extract feature points from a region except for a periphery of the region of interest in the surgical field image K(x, y) in which the region of interest is already set.

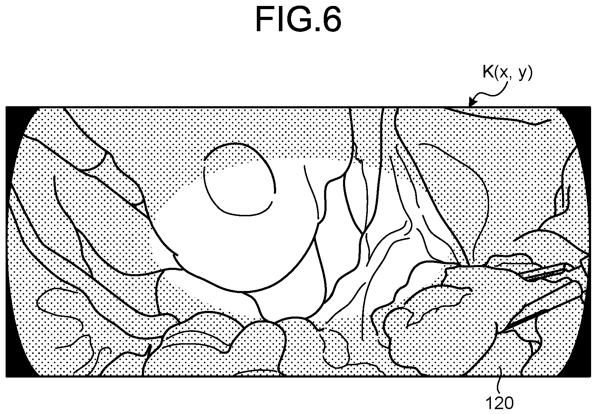

FIG. 6 is an image illustrating an example of setting of a region for extracting feature points. As illustrated in FIG. 6, the map generation unit 15 as described above sets a mask 120 in a periphery of the screen while avoiding a central part of the screen in which the frame of interest 110 is set. Then, the map generation unit 15 extracts feature points only from the inside of the set mask 120. A region of the set mask 120 is located away from the frame of interest 110 that indicates the position of the region of interest, so that it is assumed that the region is less likely to be deformed during surgery. Therefore, it is possible to stably extract feature points inside the mask 120 irrespective of a lapse of time. Further, because it is possible to stably extract feature points, it is possible to improve stability of estimation accuracy of the three-dimensional map D(X, Y, Z) and the position and the posture of the endoscope 5001.

Meanwhile, in some cases, an object, e.g., a surgical instrument such as the forceps 5023 or a finger of the surgeon, which is irrelevant to the surgical field may appear inside the mask 120 in the surgical field image K(x, y). Feature points constituting the object as described above are highly likely to irregularly move with time. In other words, it is not ensured that the feature points constituting the object as described above are stably present in the surgical field image K(x, y), which leads to an adverse effect to AF and AE. Therefore, it is preferable to extract feature points after eliminating the object as described above. To cope with this, the map generation unit 15 may have a function to eliminate, from the surgical field image K(x, y), objects, such as the surgical instruments and fingers, which are registered in advance. Meanwhile, in this case, the map generation unit 15 is one example of a detection unit according to the present disclosure.

It is sufficient to perform a process of detecting the registered objects from the surgical field image K(x, y) by, for example, using, as a template, an image that is registered in advance and searching for a region that matches the template in the surgical field image K(x, y).

### [Description of image output by medical observation system according to first embodiment]

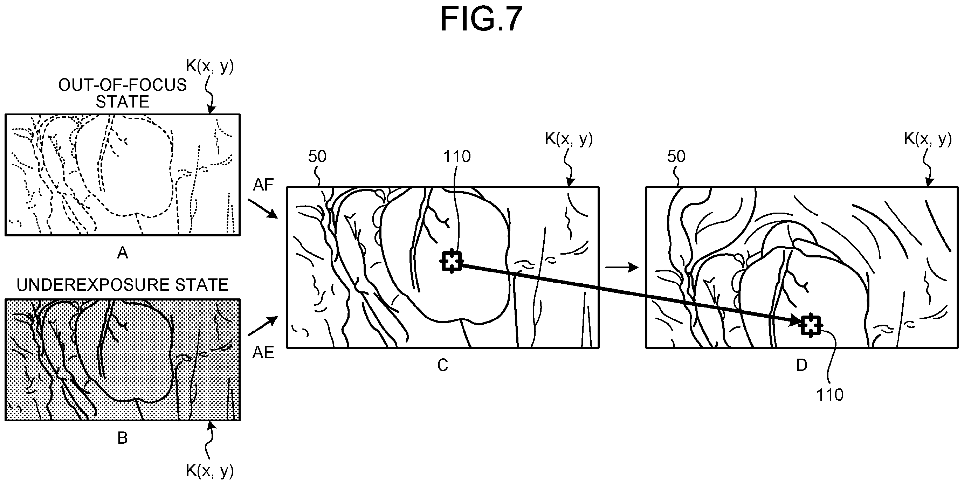

FIG. 7 is a diagram illustrating an example of the surgical field image K(x, y) that is output by the medical observation system 10a. As illustrated in FIG. 7A, in some cases, the surgical field image K(x, y) may be in what is called an out-of-focus state in which the surgical field image K(x, y) is not focused. Further, in some cases, the surgical field image K(x, y) may be in an insufficient exposure (underexposure) state as illustrated in FIG. 7B or in an excessive exposure (overexposure) state. The medical observation system 10a sets a region of interest in the surgical field image K(x, y) and adjusts the imaging parameters so as to continuously adjust the focus and the exposure with respect to the region of interest. As a result, as illustrated in FIG. 7C, the surgical field image K(x, y) in which the inside of the frame of interest 110 is focused and the inside of the frame of interest 110 is appropriately exposed is generated.

Then, the medical observation system 10a estimates a position of a region that moves with time and that is indicated by the frame of interest 110, and continuously adjusts the focus and the exposure with respect to the estimated region indicated by the frame of interest 110. Therefore, as illustrated in FIG. 7D, even if the position and the posture of the endoscope 5001 are changed and a display position of the surgical field is moved, the surgeon 5061, the scopist 5062, or the like is able to observe the set region of interest in an easily viewable manner at any time and easily carry out surgery. Conventionally, only the two-dimensional information, such as a surgical field image, is referred to, so that when, for example, an object, such as a surgical instrument or a hand of a surgeon, other than a surgical site appears in the surgical field image, the surgical site is visually lost and it becomes difficult to continuously adjust the imaging parameters in accordance with the surgical site with accuracy. Further, in a surgery place, a surgeon or a scopist frequently moves the imaging apparatus; therefore, with use of only the two-dimensional information, the surgical site is likely to be visually lost. In contrast, in the present disclosure, the region of interest is set based on the three-dimensional information, so that it is possible to continuously adjust the imaging parameters in accordance with the region of interest with high accuracy.

### [Description of function to change imaging magnification]

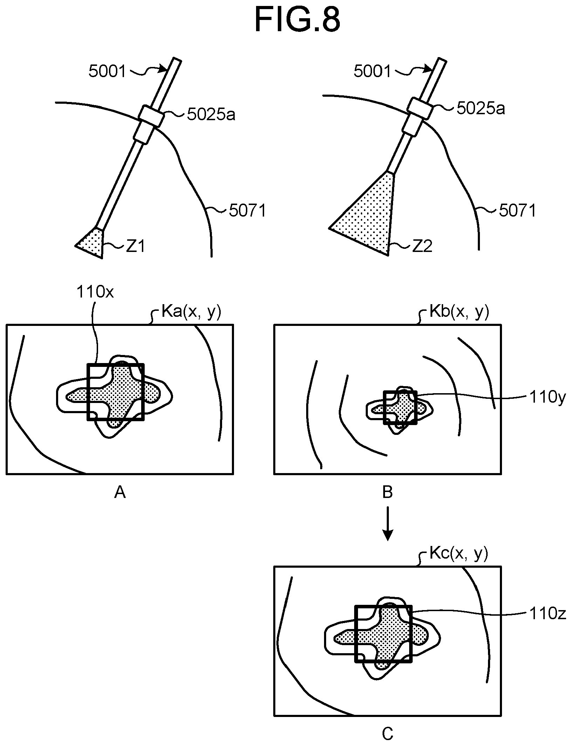

FIG. 8 is a diagram for explaining a function of the medical observation system 10a to change an imaging magnification at the time of observation in accordance with a distance to a surgical field. The medical observation system 10a is able to adjust a size of the region of interest, that is, the imaging magnification, by using information on a distance to an object (region of interest) obtained from the three-dimensional map D(X, Y, Z), and allows observation of a target object with the same size at any time.

In other words, the lens control unit 26b calculates a difference value between a distance to the region of interest that is calculated based on the information that indicates the in-focus state and that is extracted by the AF detection unit 26a, and a distance to the region of interest that is captured in previous imaging. Subsequently, the lens control unit 26b obtains a change of the magnification of the region of interest on the basis of the calculated difference value between the distances. Then, the lens control unit 26b causes the optical system 43 to control the position of a zoom lens to change the imaging magnification so that the region of interest can be observed in the same size.

For example, as illustrated in FIG. 8A, it is assumed that the endoscope 5001 that is inserted into the body cavity of the patient 5071 via the trocar 5025a captures a surgical field image Ka(x, y). Then, it is assumed that the position and the posture of the endoscope are thereafter changed and a surgical field image Kb(x, y) as illustrated in FIG. 8B is captured. At this time, a distance from the distal end of the endoscope 5001 to the surgical field increases, that is, an imaging range Z1 is changed to an imaging range Z2, so that the surgical field is observed in a reduced size (a frame of interest 110y). In this case, the lens control unit 26b is able to detect that the distance to the region of interest has increased, so that the lens control unit 26b causes the optical system 43 to control the position of the zoom lens to increase the imaging magnification.

In other words, the development processing unit 18 increases the imaging magnification of the surgical field image Kb(x, y), and generates a surgical field image Kc(x, y) as illustrated in FIG. 8C. Further, the region-of-interest setting unit 20 (setting unit) sets a frame of interest 110z by enlarging the frame of interest 110y at the same imaging magnification as the surgical field image Kc(x, y). Then, the display control unit 40 displays the frame of interest 110z in a superimposed manner on the surgical field image Kc(x, y) on the display apparatus 50. With this configuration, even if the distance from the distal end of the endoscope 5001 to the surgical field increases, it is possible to continuously observe the surgical field as the region of interest with the same size as the region of interest (the frame of interest 110x) that is observed in the surgical field image Ka(x, y).

Meanwhile, the parameter control unit 26 may set initial values of the control parameters that are used when the imaging apparatus 42a captures the surgical field image K(x, y), in accordance with the relative position that corresponds to the region of interest and that is estimated by the detection region estimation unit 22 (estimation unit), and the focus position (the three-dimensional position of the region of interest) that is adjusted by the parameter control unit 26.

### [Description of flow of process performed by medical observation system according to first embodiment]

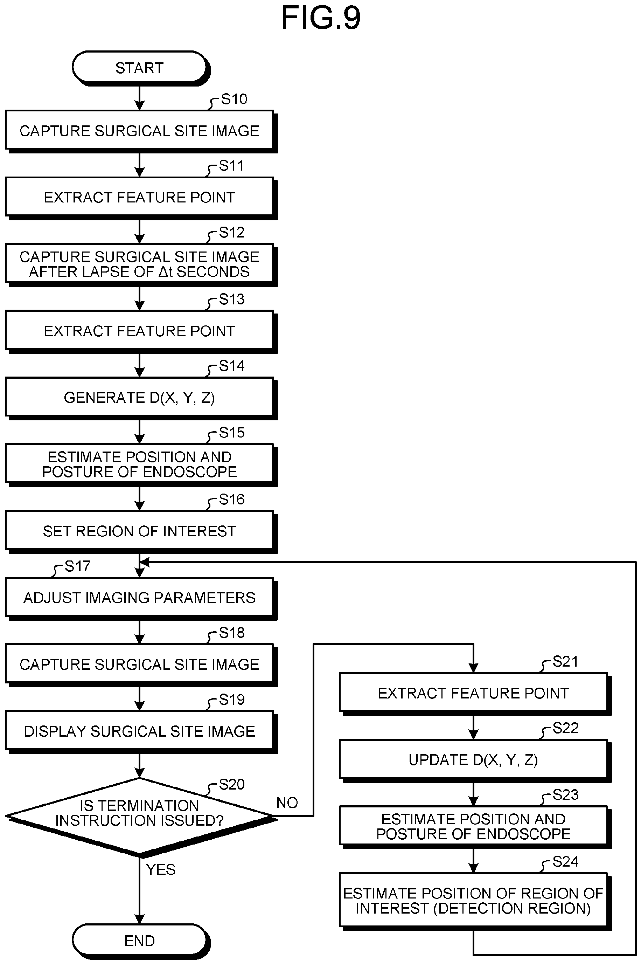

Next, the flow of the process performed by the medical observation system 10a of the first embodiment will be described. FIG. 9 is a flowchart illustrating an example of the flow of the process performed by the medical observation system 10a.

The flowchart in FIG. 8 will be described below. First, the imaging element 44a captures the surgical field image K(x, y) (Step S10) .

The map generation unit 15 extracts a feature point from the captured surgical field image K(x, y) (Step S11).

Further, the imaging element 44a captures the surgical field image K(x, y) after a lapse of Δt seconds (Step S12).

The map generation unit 15 extracts a feature point from the surgical field image K(x, y) that is captured after the lapse of Δt seconds (Step S13).

The map generation unit 15 generates the three-dimensional map D(X, Y, Z) by calculating the three-dimensional positions of the feature points (Step S14).

The subject position estimation unit 16 estimates the position and the posture of the endoscope 5001 (Step S15) .

The region-of-interest setting unit 20 sets a region of interest in the surgical field image K(x, y) (Step S16).

The parameter control unit 26 adjusts the imaging parameters (the optical parameter, the imaging parameter, the development parameter, and the light emission parameter) on the basis of pixel values of a region at the position of the region of interest in the surgical field image K(x, y) (Step S17).

The imaging element 44a captures the surgical field image K(x, y) after the lapse of Δt seconds by using the imaging parameters that are adjusted at Step S17 (Step S18) .

The display control unit 40 outputs the captured surgical field image K(x, y) to the display apparatus 50 (Step S19).

The display control unit 40 determines whether a process termination instruction is issued (Step S20). If it is determined that the termination instruction is issued (Step S20: Yes), the medical observation system 10a terminates the process in FIG. 6. In contrast, if it is not determined that the termination process is issued (Step S20: No), the process proceeds to Step S21. Meanwhile, the process termination instruction is issued by detecting operation of turning off a power switch (not illustrated) of the camera control unit 12a, for example.

If it is determined as No at Step S20, the map generation unit 15 extracts a feature point from the surgical field image K(x, y) that is captured after the lapse of Δt seconds (Step S21).

The map generation unit 15 calculates three-dimensional positions of the feature points, to thereby update the three-dimensional map D(X, Y, Z) that is generated at Step S14 (Step S22).

The subject position estimation unit 16 estimates the position and the posture of the endoscope 5001 (Step S23) .

The detection region estimation unit 22 estimates a position of the region of interest (detection region) in the surgical field image K(x, y) that is captured after the lapse of Δt seconds at Step S18 (Step S24). Thereafter, the process returns to Step S17.

### [Description of operation and effects of first embodiment]

As described above, according to the medical observation system 10a of the first embodiment, the three-dimensional information generation unit 14 generates the three-dimensional map D(X, Y, Z) (three-dimensional information) of the surgical field from the surgical field image K(x, y) that is captured by the imaging apparatus 42a. Further, the region-of-interest setting unit 20 (setting unit) sets at least one region of interest in the surgical field image K(x, y) that is captured at a predetermined timing. The detection region estimation unit 22 (estimation unit) estimates the relative position corresponding to the region of interest from the surgical field image K(x, y) that is captured at a certain timing that is different from the predetermined timing, on the basis of the three-dimensional map D(X, Y, Z) and the information on the region of interest that is set by the region-of-interest setting unit 20. Then, the parameter control unit 26 (adjustment unit) adjusts the imaging parameters of the imaging apparatus 42a and the light source apparatus 60 that is a peripheral device of the imaging apparatus 42a on the basis of three-dimensional information and pixel values of the surgical field image K(x, y) corresponding to the relative position that corresponds to the region of interest and that is estimated by the detection region estimation unit 22, and causes the surgical field image K(x, y) to be captured. Then, the display control unit 40 displays the captured surgical field image K(x, y). Therefore, even if the position and the posture of the endoscope 5001 in which the imaging apparatus 42a is mounted are changed, it is possible to continuously adjust the imaging parameters related to the AF function, the AE function, and the like in accordance with the region of interest, such as a surgical site.

Furthermore, according to the medical observation system 10a of the first embodiment, the imaging apparatus 42a includes the single imaging element 44a, and the three-dimensional information generation unit 14 generates the three-dimensional map D(X, Y, Z) (three-dimensional information) of the surgical field on the basis of at least the two surgical field images K(x, y) that are captured at different times by the imaging apparatus 42a. Therefore, with use of the imaging apparatus 42a having a simple structure including only a monocular camera, it is possible to continuously observe the surgical field image K(x, y) in which the region of interest, such as the surgical site, is focused and the region of interest is captured with easily viewable brightness.

Moreover, according to the medical observation system 10a of the first embodiment, the three-dimensional information generation unit 14 generates the three-dimensional information on the feature points that are extracted from the entire range or an arbitrary range in the surgical field image K(x, y). Therefore, it is possible to extract feature points from the entire surgical field image K(x, y) before setting the region of interest, and generates the three-dimensional map D(X, Y, Z) based on the three-dimensional positions of as many feature points as possible. Further, after the region of interest is set, feature points are not extracted from the region of interest that largely varies due to surgery and a region near the region of interest, so that it is possible to update the three-dimensional map D(X, Y, Z) on the basis of three-dimensional positions of stable feature points that are less likely to move with time.

Furthermore, according to the medical observation system 10a of the first embodiment, the parameter control unit 26 (adjustment unit) adjusts, as the control parameters, the optical parameters that define the state of the optical system of the imaging apparatus 42a, in accordance with the pixel values of the region corresponding to the estimated position of the region of interest that is estimated by the detection region estimation unit 22 (estimation unit) in the surgical field image K(x, y). Therefore, even if the position of the region of interest is moved, it is possible to obtain the surgical field image K(x, y) in which the region of interest is focused.

Moreover, according to the medical observation system 10a of the first embodiment, the parameter control unit 26 (adjustment unit) adjusts, as the control parameters, the imaging parameters that defines an exposure condition of the imaging apparatus 42a, in accordance with the pixel values of the region corresponding to the estimated position of the region of interest that is estimated by the detection region estimation unit 22 (estimation unit) in the surgical field image K(x, y). Therefore, even if the position of the region of interest is moved, it is possible to obtain the surgical field image K(x, y) that is exposed such that the region of interest can easily be viewed.

Furthermore, according to the medical observation system 10a of the first embodiment, the parameter control unit 26 (adjustment unit) adjusts, as the control parameters, the development parameters for defining the development condition of the imaging apparatus 42a, in accordance with the pixel values of the region corresponding to the estimated position of the region of interest that is estimated by the detection region estimation unit 22 (estimation unit) in the surgical field image K(x, y). Therefore, even if the position of the region of interest is moved, it is possible to obtain the surgical field image K(x, y) that is subjected to exposure correction such that the region of interest can easily be viewed.

Moreover, according to the medical observation system 10a of the first embodiment, the parameter control unit 26 (adjustment unit) adjusts, as the control parameters, light emission parameters for defining a light emission state of the light source apparatus 60 that applies illumination light to the imaging range of the imaging apparatus 42a, in accordance with the pixel values of the region corresponding to the estimated position of the region of interest that is estimated by the detection region estimation unit 22 (estimation unit) in the surgical field image K(x, y). Therefore, even if the position of the region of interest is moved, it is possible to obtain the surgical field image K(x, y) that is illuminated such that the region of interest can easily be viewed.

Furthermore, according to the medical observation system 10a of the first embodiment, the map generation unit 15 (detection unit) detects an object that is registered in advance from the surgical field image K(x, y). Then, the region-of-interest setting unit 20 (setting unit) extracts feature points from a region except for objects detected by the map generation unit 15 (detection unit) in the surgical field image K(x, y). Therefore, feature points of the objects, such as the forceps 5023 and fingers, are not extracted, so that it is possible to prevent an adverse effect on AF and AE.

Moreover, according to the medical observation system 10a of the first embodiment, the parameter control unit 26 (adjustment unit) changes the imaging magnification of the surgical field image K(x, y) captured by the imaging apparatus 42a in accordance with the three-dimensional position of the region of interest, where the three-dimensional position is based on the relative position that corresponds to the region of interest and that is estimated by the detection region estimation unit 22 (estimation unit) and the focus position (the three-dimensional position of the region of interest) that is adjusted by the parameter control unit 26. Then, the region-of-interest setting unit 20 (setting unit) changes the size of the region of interest in accordance with the imaging magnification. Therefore, even if the position and the posture of the endoscope 5001 are changed, it is possible to continuously observe the region of interest with the same size in the surgical field image K(x, y).

Furthermore, according to the medical observation system 10a of the first embodiment, the parameter control unit 26 (adjustment unit) sets initial values of the control parameters that are used when the imaging apparatus 42a captures the surgical field image K(x, y), in accordance with the relative position that corresponds to the region of interest and that is estimated by the detection region estimation unit 22 (estimation unit) and the focus position (the three-dimensional position of the region of interest) that is adjusted by the parameter control unit 26. Therefore, even if the position and the posture of the endoscope 5001 are changed, it is possible to promptly adjust the control parameters when the region of interest in the surgical field image K(x, y) is captured.

Moreover, according to the medical observation system 10a of the first embodiment, the imaging apparatus 42a is mounted on the endoscope 5001. Therefore, when surgery or the like is performed using the endoscope 5001, it is possible to continuously adjust the focus and the exposure with respect to the affected area.

Furthermore, according to the camera control unit 12a (medical observation apparatus) of the first embodiment, the three-dimensional information generation unit 14 generates the three-dimensional map D(X, Y, Z) (three-dimensional information) from at least the two surgical field images K(x, y) in which the surgical field is captured at different positions. Further, the region-of-interest setting unit 20 (setting unit) sets at least one region of interest in the surgical field image K(x, y) that is captured at a predetermined timing. The detection region estimation unit 22 (estimation unit) estimates the relative position corresponding to the region of interest from the surgical field image K(x, y) that is captured at a certain timing different from the predetermined timing, on the basis of the three-dimensional map D(X, Y, Z) and the position of the region of interest that is set by the region-of-interest setting unit 20. Then, the parameter control unit 26 (adjustment unit) adjusts the imaging parameters of the imaging apparatus 42a and the light source apparatus 60 that is a peripheral device of the imaging apparatus 42a on the basis of the three-dimensional map D(X, Y, Z) and the relative position of the region of interest, and causes the surgical field image K(x, y) to be captured, and thereafter, the display control unit 40 displays the captured surgical field image K(x, y). Therefore, even if the endoscope 5001 on which the imaging apparatus 42a is mounted is moved, it is possible to continuously adjust the imaging parameters related to the AF function, the AE function, and the like in accordance with the the region of interest, such as a surgical site.

Meanwhile, in the medical observation system 10a, the endoscope 5001 in which the imaging apparatus 42a is arranged may include an acceleration sensor, such as a gyro sensor. It is possible to measure the position and the posture of the endoscope 5001 from an output of the acceleration sensor, so that it is possible to measure the position and the posture of the endoscope 5001 without causing the imaging apparatus 42a to capture two images at different times, and it is possible to accordingly estimate the position of the region of interest.

### Second Embodiment

A configuration of the medical observation system 10a is not limited to the configuration described in the first embodiment, and various modifications may be made. Different embodiments of the medical observation system will be sequentially described below.

In the first embodiment, it is explained that the medical observation system 10a is configured such that the imaging apparatus 42a includes the single imaging element 44a. However, the configuration of the imaging apparatus is not limited to this example.

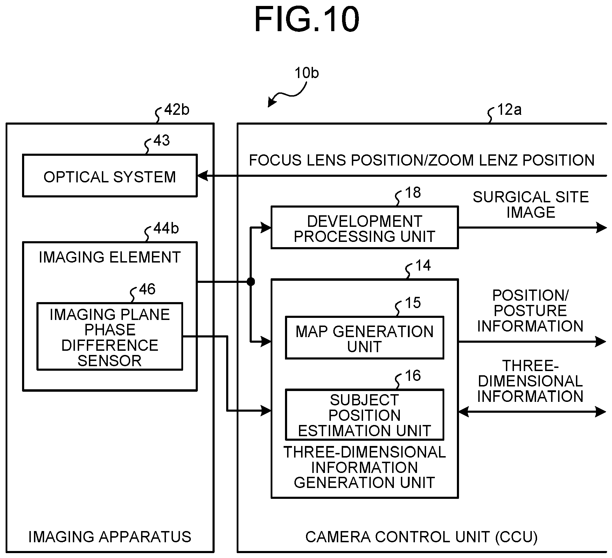

FIG. 10 is a diagram illustrating an example of a schematic configuration of a medical observation system 10b in which an imaging apparatus 42b includes an imaging element 44b that includes an imaging plane phase difference sensor 46. Meanwhile, in FIG. 10, some of the components illustrated in FIG. 2 are omitted, and the omitted components have the same configurations as those of FIG. 2 unless otherwise specified.

The imaging plane phase difference sensor 46 is configured such that pixels that measure a distance are arranged in a discrete manner inside the imaging element 44b. With use of the medical observation system 10b configured as illustrated in FIG. 10, the map generation unit 15 is able to extract depth information (distance information) with respect to the captured object 100, from imaging plane phase difference information that is output by the imaging plane phase difference sensor 46. Therefore, it is possible to effectively use the SLAM technique. Meanwhile, the imaging plane phase difference sensor 46 is able to obtain the depth information from only a single captured image.

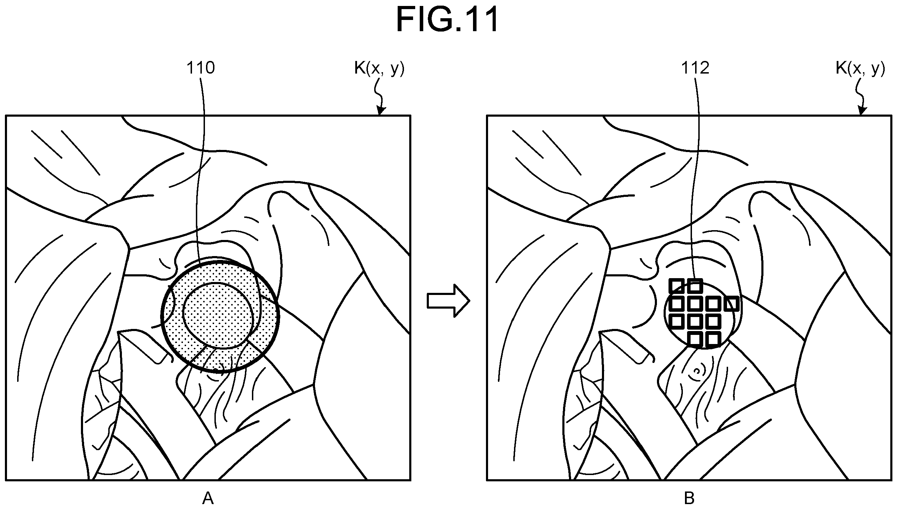

FIG. 11 is a diagram illustrating an example of a display mode of the surgical field image K(x, y) when the imaging plane phase difference sensor is used. As illustrated in FIG. 11A, the frame of interest 110 is superimposed on the region of interest of the surgical field image K(x, y). In this case, the imaging plane phase difference sensor 46 measures a distance to the surgical field inside the frame of interest 110. A distance measurement result is displayed by a plurality of indicators 112 indicating in-focus positions as illustrated in FIG. 11B.

As described above, according to the second embodiment, it is possible to obtain the depth information from the single captured surgical field image K(x, y), so that even if the object is moving, it is possible to control AF and AE with high accuracy by measuring the three-dimensional position of the object with high accuracy.

### Third Embodiment

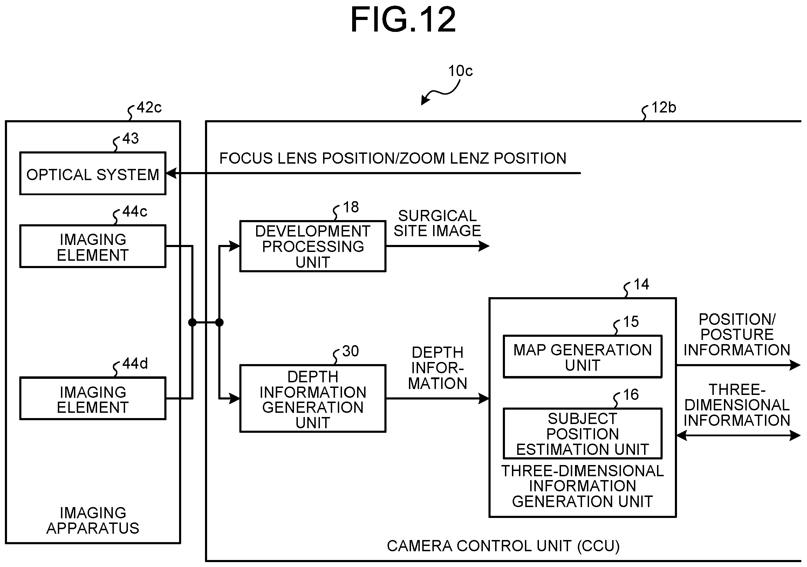

FIG. 12 is a diagram illustrating an example of a schematic configuration of a medical observation system 10c in which an imaging apparatus 42c includes two imaging elements 44c and 44d. Meanwhile, the two imaging elements 44c and 44d are arranged such that a relative relationship that is determined in advance is maintained, and capture different portions of an affected area such that the portions partly overlap with each other. More specifically, the imaging elements 44c and 44d acquire image signals for a right eye and a left eye corresponding to a stereoscopic view. Meanwhile, in FIG. 12, some of the components illustrated in FIG. 2 are omitted, and the omitted components have the same configurations as those of FIG. 2 unless otherwise specified.

Further, in the medical observation system 10c, a camera control unit 12b includes a depth information generation unit 30 in addition to the components illustrated in FIG. 2. The depth information generation unit 30 performs matching between two surgical field images that are respectively captured by the two imaging elements 44c and 44d, and generates the depth information.

With use of the medical observation system 10c configured as illustrated in FIG. 12, the map generation unit 15 is able to generate the three-dimensional map D(X, Y, Z) using the SLAM technique, on the basis of the depth information that is generated by the depth information generation unit 30 and the surgical field images that are respectively captured by the imaging elements 44c and 44d. Furthermore, the two imaging elements 44c and 44d are able to perform imaging simultaneously, so that it is possible to obtain the depth information from the two images that are captured by single imaging. Therefore, even if the object is moving, it is possible to measure the three-dimensional position of the object with high accuracy.

As described above, according to the third embodiment, the imaging apparatus 42c includes the two imaging elements 44c and 44d that capture different ranges that partly overlap with each other, and the three-dimensional information generation unit 14 generates the three-dimensional information on the surgical field on the basis of the two surgical field images K(x, y) that are captured by the imaging apparatus 42c at the same time. Therefore, it is possible to obtain the depth information from the two surgical field images K(x, y) that are obtained by single imaging, so that even if the surgical field is moving, it is possible to measure the three-dimensional position of the surgical field with high accuracy.

### Fourth Embodiment

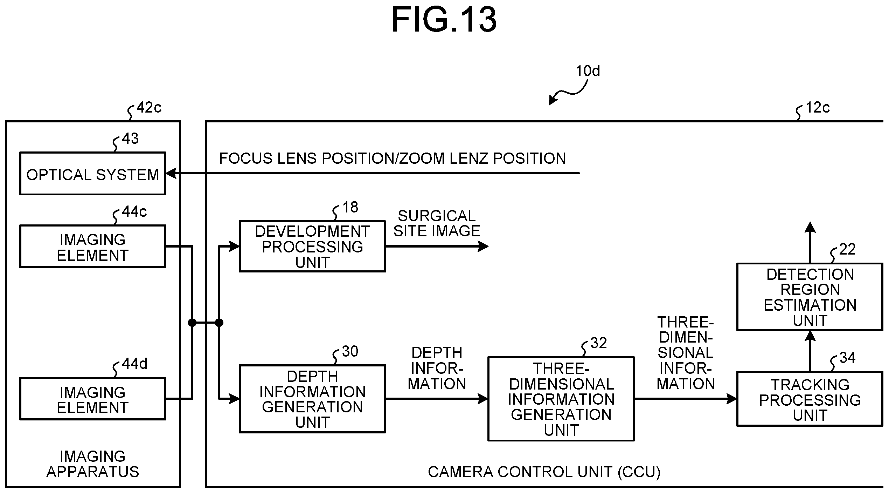

FIG. 13 is a diagram illustrating an example of a schematic configuration of a medical observation system 10d in which the imaging apparatus 42c includes two imaging elements and a camera control unit 12c includes a tracking processing unit 34. Meanwhile, in FIG. 13, some of the components illustrated in FIG. 2 are omitted, and the omitted components have the same configurations as those of FIG. 2 unless otherwise specified.

The camera control unit 12c of the medical observation system 10d includes the depth information generation unit 30, a three-dimensional information generation unit 32, the tracking processing unit 34, and a zoom region calculation unit 36.

The three-dimensional information generation unit 32 is provided instead of the three-dimensional information generation unit 14, and generates the three-dimensional information on the surgical field image K(x, y) on the basis of the depth information that is generated by the depth information generation unit 30. The tracking processing unit 34 is provided instead of the three-dimensional map data storage unit 24, and calculates a difference in the position and the posture of the imaging apparatus 42c by adopting the iterative closest point (ICP) method or the like for overlapping two point groups, on the basis of three-dimensional information on a previous frame and three-dimensional information on a current frame. The detection region estimation unit 22 calculates a position of a detection region in the surgical field image K(x, y) on the basis of a difference value in the position and the posture of the imaging apparatus 42c calculated by the tracking processing unit 34. Further, the parameter control unit 26 as described above (FIG. 2) calculates the control parameters for performing imaging with focus on the calculated detection region and with brightness with which the detection region can easily be viewed. Furthermore, the parameter control unit 26 causes the imaging apparatus 42c to capture the surgical field image K(x, y) using the calculated control parameters.

As described above, according to the fourth embodiment, it is possible to stably track (trace) the region of interest in the surgical field image K(x, y) independent of movement of the imaging apparatus 42c.

### Fifth Embodiment

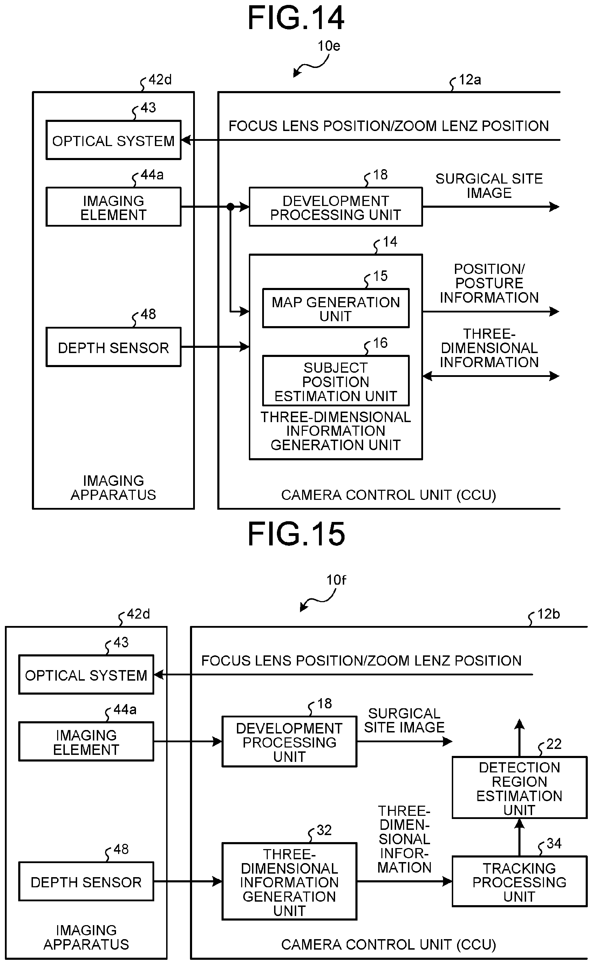

FIG. 14 is a diagram illustrating an example of a schematic configuration of a medical observation system 10e in which an imaging apparatus 42d includes the imaging element 44a and a depth sensor 48. Meanwhile, in FIG. 14, some of the components illustrated in FIG. 2 are omitted, and the omitted components have the same configurations as those of FIG. 2 unless otherwise specified.

The depth sensor 48 is what is called a three-dimensional (3D) sensor that measures a distance to an object. The depth sensor 48 is what is called a time to flight (ToF) sensor that measures the distance to the object by receiving reflected light, such as infrared light, of light applied to the object, and measures a flight time of the light. Furthermore, the depth sensor 48 is realized by what is called a pattern projection method (structured light) that measures the distance to the object by capturing an image of projection light that has a plurality of different geometric patterns and that is applied to the object.

The map generation unit 15 extracts the depth information (distance information) with respect to the captured object 100, on the basis of the surgical field image K(x, y) that is captured by the imaging element 44a and the distance that is output by the depth sensor 48. More specifically, the map generation unit 15 calculates a pixel is in the surgical field image K(x, y) captured by the imaging element 44a and that corresponds to a point measured by the depth sensor. Furthermore, the map generation unit 15 generates the three-dimensional map D(X, Y, Z) (three-dimensional information) of the surgical field. Therefore, it is possible to effectively use the SLAM technique.

As described above, according to the fifth embodiment, the imaging apparatus 42d includes the single imaging element 44a and the depth sensor 48 (distance measurement apparatus) that measures a distance to a target object, and the three-dimensional information generation unit 14 generates the three-dimensional map D(X, Y, Z) (three-dimensional information) of the surgical field on the basis of the image that is captured by the imaging element 44a and the distance that is measured by the depth sensor 48. Therefore, it is possible to easily and reliably measure the distance to the surgical field.

### Sixth Embodiment

FIG. 15 is a diagram illustrating an example of a schematic configuration of a medical observation system 10f in which the imaging apparatus 42d includes the imaging element 44a and the depth sensor 48, and a camera control unit 12d includes the tracking processing unit 34. Meanwhile, in FIG. 15, some of the components illustrated in FIG. 2 are omitted, and the omitted components have the same configurations as those of FIG. 2 unless otherwise specified.

The camera control unit 12d of the medical observation system 10f includes the three-dimensional information generation unit 32, the tracking processing unit 34, and the zoom region calculation unit 36.

The three-dimensional information generation unit 32 is provided instead of the three-dimensional information generation unit 14, and the depth sensor 48 obtains a moving state of the surgical field by performing matching between two pieces of distance information measured from different positions (for example, distance images in which pixel values corresponding to the distances to the object are stored). The tracking processing unit 34 is provided instead of the three-dimensional map data storage unit 24, and calculates a difference in the position and the posture of the imaging apparatus 42c on the basis of the moving state of the surgical field as described above. The detection region estimation unit 22 calculates the position of the detection region in the surgical field image K(x, y) on the basis of the difference value of the position and the posture of the imaging apparatus 42d calculated by the tracking processing unit 34. Further, the parameter control unit 26 as described above (FIG. 2) calculates the control parameters for performing imaging with focus on the calculated detection region and with brightness with which the detection region can easily be viewed. Furthermore, the parameter control unit 26 causes the imaging apparatus 42d to capture the surgical field image K(x, y) using the calculated control parameters.

As described above, according to the sixth embodiment, it is possible to stably track (trace) the region of interest in the surgical field image K(x, y) independent of movement of the imaging apparatus 42d.

### Seventh Embodiment

FIG. 16 is a diagram illustrating an example in which a plurality of frames of interest 110a and 110b are set in the surgical field image K(x, y).

As illustrated in FIG. 16, the region-of-interest setting unit 20 (FIG. 2) may set a plurality of regions of interest in the surgical field image K(x, y). For example, if it is necessary to pay attention to a plurality of affected areas, the region-of-interest setting unit 20 sets the frames of interest 110a and 110b indicating the respective regions of interest, on the basis of an instruction issued by the scopist 5062. The parameter control unit 26 performs AF control such that the regions of interest respectively including the set frames of interest 110a and 110b are focused. Further, the AE control and the light source control are performed such that the regions of interest including the frames of interest 110a and 110b have brightness with which observation can easily be performed. At this time, the parameter control unit 26 determines appropriate control parameters such that both of the frames of interest 110a and 110b are focused and both of the frames of interest 110a and 110b have brightness with which observation can easily be performed, by using the distances to the regions in which the frames of interest 110a and 110b are set, for example. Then, the display control unit 40 outputs the surgical field image K(x, y) that is captured based on the control parameters adjusted by the parameter control unit 26 to the display apparatus 50.

Meanwhile, as a modification of the present embodiment, it may be possible to set the frame of interest 110a that is one of the frames of interest 110a and 110b as a region that is to be focused, and set the other frame of interest 110b as a region that is not to be focused. In this manner, by setting a region to be eliminated from a focus target, it becomes not necessary to perform an unnecessary search for a position that is focused when, for example, the lens control unit 26b performs arithmetic processing for contrast AF or the like, so that it is possible to increase a focus speed. Meanwhile, it is preferable to display the frame of interest 110a indicating the region that is to be focused and the frame of interest 110b indicating the region that is not to be focused, in different colors or different modes in order to improve distinguishability.

As described above, according to the seventh embodiment, when a plurality of regions to which attention is paid are present in the surgical field, the region-of-interest setting unit 20 sets a plurality of regions of interest. Therefore, it is possible to adjust the control parameters for the plurality of regions of interest.

### Eighth Embodiment

FIG. 17 is a diagram illustrating an example of a display mode in which a region in a predetermined distance range is displayed in a highlighted manner in the surgical field image K(x, y).

As illustrated in FIG. 17, when setting a region of interest, the region-of-interest setting unit 20 gives a predetermined color to the region in the predetermined distance range in the surgical field image K(x, y). FIG. 17 illustrates an example in which a region R1 at a shorter distance than a distance d1 and a region R2 at a farther distance than a distance d2 are displayed in different colors. Meanwhile, this is a process of limiting a range of a distance to the region of interest between the distance d1 to the distance d2 to make it possible to easily set a region of interest.

Values of the distance d1 and the distance d2 may be set by, as illustrated in FIG. 17 for example, causing the region-of-interest setting unit 20 to display a distance scale in the vicinity of the surgical field image K(x, y) and allowing the scopist 5062 to operate an input device, such as a mouse or a touch panel. Then, the region-of-interest setting unit 20 colors and displays the region R1 and the region R2 on the surgical field image K(x, y) in real time, in accordance with the set values of the distance d1 and the distance d2. At this time, the operator sets the distance d1 or the distance d2 by performing pointing, by the input device, at a position of a distance to be set on the distance scale. Then, the operator drags the input device toward a far direction or a near direction on the distance scale while keeping performing pointing on the input device. The region-of-interest setting unit 20 detects the drag operation, and, as illustrated in FIG. 17, displays colors to be added to the dragged distance ranges. With use of a graphical user interface (GUI) as described above, the operator is able to easily recognize the region corresponding to the distance range that the operator has set in the surgical field image K(x, y). Meanwhile, the method of displaying the set distance range on the distance scale is not limited to the method as illustrated in FIG. 17, and other display modes may be adopted as long as the set distance range is clearly indicated.

The display control unit 40 outputs the surgical field image K(x, y) in which the region R1 and the region R2 are displayed with colors to the display apparatus 50. Then, the scopist 5062 sets the region of interest in accordance with the procedure as described above (see FIG. 4) while viewing the surgical field image K(x, y) in which the region R1 and the region R2 are colored.

As described above, according to the eighth embodiment, the region-of-interest setting unit 20 (setting unit) additionally has a function to specify a distance range in which a region of interest is present, and causes a region of interest to be set in the specified distance range. Therefore, the scopist 5062 is able to more easily set the region of interest.

### Ninth Embodiment

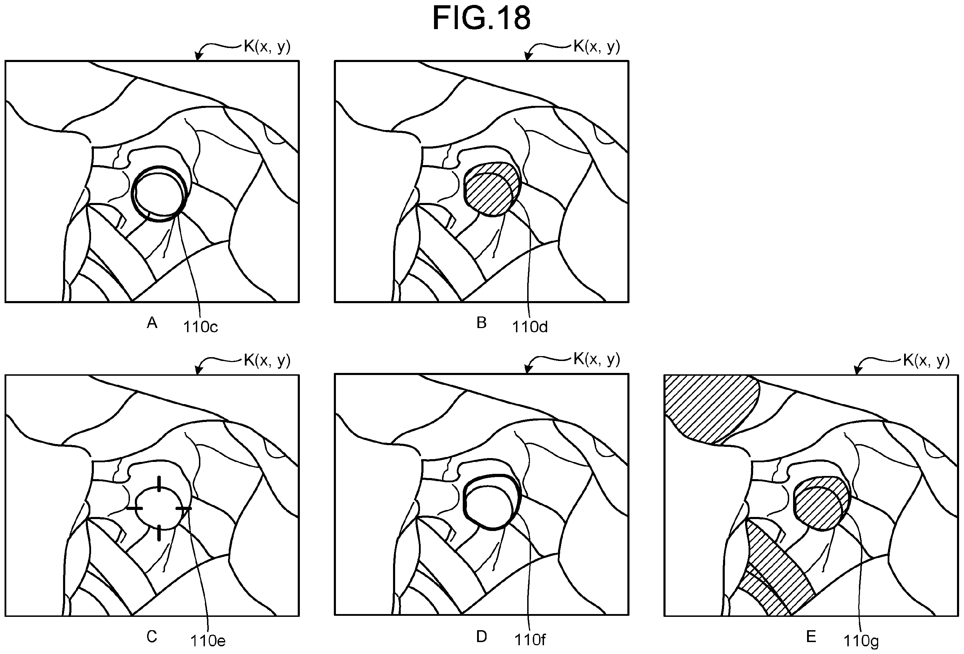

FIG. 18 is a diagram illustrating an example of display modes of frames of interest 110c to 110g that are set in the surgical field image K(x, y).

The display mode of the frame of interest is not limited to a rectangular frame as illustrated in FIG. 4. FIG. 18A illustrates an example in which the frame of interest 110c is displayed as a circular region. FIG. 18B illustrates an example in which the frame of interest 110d is indicated by a colored (highlighted) closed region. FIG. 18C illustrates an example in which the frame of interest 110e is indicated by a symbol. FIG. 18D illustrates an example in which the frame of interest 110f is indicated by a closed curve. FIG. 18E illustrates an example in which regions located at the same distance as the positions at which the frame of interest 110g and the frame of interest 110g are set are displayed with colors. In particular, according to the display mode as illustrated in FIG. 18E, the scopist 5062 is able to recognize that different regions are present at the same distance as the region of interest. Therefore, it is possible to more carefully hold the endoscope 5001 so as to prevent a tracking failure of the region of interest when the endoscope 5001 is erroneously oriented toward a different region.

Meanwhile, it is sufficient for the scopist 5062 to set a display mode to be adopted for the frame of interest, in the region-of-interest setting unit 20 in advance. Further, it is sufficient to adopt the same method as illustrated in FIG. 4 or FIG. 5 as the method of setting the frames of interest 110c to 110g. It may be possible to directly set the position of the frame of interest on the screen by using an input device, such as a touch panel or a mouse, instead of setting the frame of interest after moving the region of interest to the center of the screen. In particular, as illustrated in FIG. 18B, FIG. 18D, and FIG. 18E, if an arbitrary shape of the frame of interest is set as a closed region, it is effective to directly set the position and the shape of the frame of interest on the surgical field image K(x, y) displayed on the display apparatus 50 as illustrated in FIG. 5.

As described above, according to the ninth embodiment, it is possible to display, in the set region of interest, the frames of interest 110c to 110g in the modes that can easily be viewed by the operator.

### Tenth Embodiment

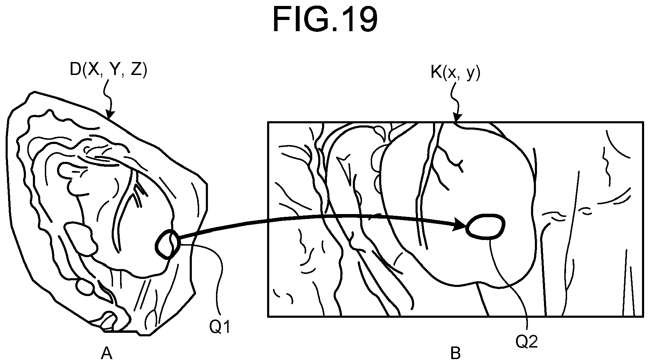

FIG. 19 is a diagram for explaining a function to detect a portion with intensive reflected light in the surgical field image K(x, y). The abdominal cavity and the organs are filled with liquid, such as ascites, mucus, and blood. The liquid as described above has high specular reflection property when illumination light is applied from outside. If the imaging element 44a receives the specular reflected light generated as described above, a pixel value of the pixel that has received the light may overflow. If the overflow as described above occurs, it becomes difficult to distinguish the brightness, the color, the shape, and the like of the surgical field image K(x, y), so that the image may become an undesirable image for observing the surgical field. Therefore, in the medical observation system 10a, it is preferable to adopt an observation layout in which the imaging element 44a does not receive specular reflected light.

A traveling direction of the specular reflected light depends on a normal direction of a stereoscopic object in the abdominal cavity. Further, the normal direction of the stereoscopic object inside the abdominal cavity is generally distributed in various directions, so that it is not realistic to realize the observation layout in which the imaging element 44a does not receive specular reflected light. To cope with this, in the present embodiment, positions at which specular reflected light is likely to occur are predicted in advance, and the positions are prevented from being set as regions of interest that are adopted as observation targets.

In the present embodiment, the region-of-interest setting unit 20 of the medical observation system 10a described in the first embodiment is further provided with a function to predict a position at which specular reflected light occurs in the surgical field image K(x, y). In other words, the region-of-interest setting unit 20 checks the three-dimensional map D(X, Y, Z), which is generated by the map generation unit 15 and illustrated in FIG. 19A, with a set position of the light source apparatus 60, and predicts a position at which specular reflected light emitted from the light source apparatus 60 occurs in the surgical field image K(x, y) illustrated in FIG. 19B.

Specifically, the map generation unit 15 calculates a three-dimensional normal direction at a point (X, Y, Z) on the basis of three-dimensional positions of adjacent points in the three-dimensional map D(X, Y, Z). Then, the region-of-interest setting unit 20 checks the normal direction that is calculated by the map generation unit 15 (the three-dimensional information generation unit 14) with the set position of the light source apparatus 60, and calculates a position at which specular reflected light of light rays emitted by the light source apparatus 60 arrives at the imaging element 44a. The arrival position of the specular reflected light may be calculated by using a ray tracing method. Through the process as described above, for example, it is possible to predict that specular reflected light in the region Q1 in FIG. 19A arrives at the region Q2 in the surgical field image K(x, y) in FIG. 19B.

The region-of-interest setting unit 20 further sets a mask in the region Q2 that is the arrival position of the specular reflected light. Then, the region-of-interest setting unit 20 prevents the region of interest from being set in the region Q2.

As described above, according to the tenth embodiment, the region-of-interest setting unit 20 (setting unit) predicts a direction of specular reflected light of illumination light emitted by the light source apparatus 60, on the basis of the three-dimensional map D(X, Y, Z) (the three-dimensional information) of the surgical field generated by the three-dimensional information generation unit 14. Further, if it is determined that the specular reflected light that travels in the predicted direction is to be captured by the imaging apparatus 42a, the region-of-interest setting unit 20 eliminates the region, in which the specular reflected light is captured, from a setting target of the region of interest. Therefore, the region-of-interest setting unit 20 sets a region of interest at a position at which the specular reflected light does not arrive, so that it is impossible to obtain appropriate exposure by performing AE control on the basis of pixel values in the region of interest.

Meanwhile, in the present embodiment, if the specular reflected light is captured by the imaging apparatus 42a, it may be possible to adjust the control parameters and the light source control parameters for AE in order to prevent pixel values from overflowing due to the observed specular reflected light.

### Eleventh Embodiment

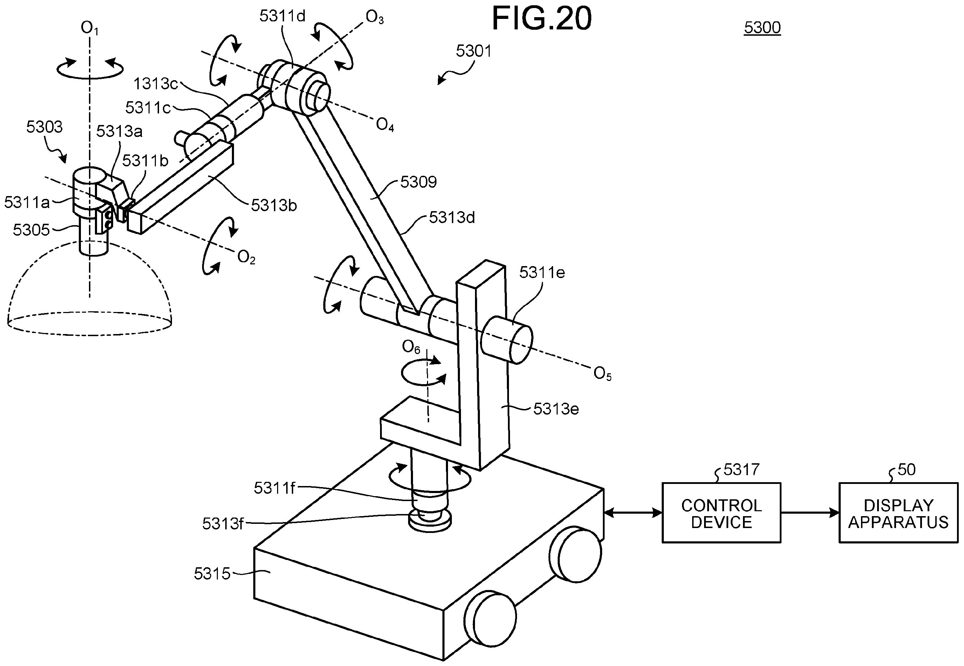

FIG. 20 is a diagram illustrating an example of a schematic configuration of a microscope surgical system 5300 to which the technique according to the present disclosure is applicable. With reference to FIG. 20, the microscope surgical system 5300 includes a microscope device 5301, a control device 5317, and the display apparatus 50. Meanwhile, in the following description of the microscope surgical system 5300, a "user" indicates an arbitrary medical staff, such as a surgeon and an assistant, who uses the microscope surgical system 5300.

The microscope device 5301 includes a microscope unit 5303 used to observe an observation target (a surgical site of a patient) in an enlarged manner, an arm section 5309 that supports the microscope unit 5303 at a distal end thereof, and a base section 5315 that supports a proximal end of the arm section 5309.

The microscope unit 5303 includes a tubular part 5305 that has an approximately cylindrical shape, and an imaging unit (not illustrated) that is arranged inside the tubular part 5305. The microscope unit 5303 is an electronic imaging type microscope unit (what is called a video microscope unit) that electronically captured an image by using the imaging unit. Meanwhile, the imaging unit is one example of the imaging apparatus according to the present disclosure.

A cover glass for protecting the internally-arranged imaging unit is arranged on an opening surface at a lower end of the tubular part 5305. Light from an observation target (hereinafter, also referred to as observation light) passes through the cover glass and enters the imaging unit arranged inside the tubular part 5305. Meanwhile, it may be possible to arrange a light source including a light emitting diode (LED) or the like inside the tubular part 5305, and it may be possible to apply light from the light source to the observation target via the cover glass at the time of imaging.

The imaging unit includes an optical system that collects observation light, and an imaging element that receives the observation light collected by the optical system. The optical system includes a plurality of lenses including a zoom lens and a focus lens, and optical characteristics of the optical system are adjusted such that the observation light is collected so as to form an image on a light-receiving surface of the imaging element. The imaging element receives the observation light, performs photoelectric conversion on the observation light, and generates a signal corresponding to the observation light, that is, an image signal corresponding to an observation image. As the imaging element, for example, an element that has Bayer arrangement and is capable of capturing a color image may be used. The imaging element may be various well-known imaging elements, such as a CMOS image sensor or a CCD image sensor. The image signal generated by the imaging element is transmitted, as RAW data, to the control device 5317. Here, it may be possible to transmit the image signal by optical communication as a preferable example. In a surgery place, a surgeon performs surgery while observing a state of an affected area by using the captured image; therefore, to more stably and reliably perform surgery, it is desired to display a video of the surgical field in real time as much as possible. By transmitting the image signal by the optical communication, it becomes possible to display the captured image at low latency.

Meanwhile, the imaging unit may be provided with a driving function to move the zoom lens and the focus lens of the optical system along the optical axis. By appropriately moving the zoom lens and the focus lens by the driving mechanism, an imaging magnification of the captured image and a focal length at the time of imaging may be adjusted. Further, the imaging unit may be provided with various functions, such as an AE function and an AF function, which may be included in a general electronic imaging type microscope unit.

Furthermore, the imaging unit may be configured as what is called a single-chip imaging unit that includes a single imaging element, or what is called a multiple-chip imaging unit that includes a plurality of imaging elements. If the imaging element is configured as the multiple-chip imaging unit, for example, each of the imaging elements may generate an image signal corresponding to each of RGB, and a color image may be obtained by combining the image signals. Alternatively, the imaging unit may be configured to include a pair of imaging elements for acquiring image signals for a right eye and a left eye corresponding to stereoscopic view (3D-display). By performing 3D-display, the surgeon is able to more accurately recognize a depth of living tissue in the surgical field. Meanwhile, if the imaging unit is configured as a multiple-chip system, a plurality of optical systems may be arranged for the respective imaging elements.

The arm section 5309 is configured such that a plurality of links (a first link 5313a to a sixth link 5313f) are rotatably connected to a plurality of joint sections (a first joint section 5311a to a sixth joint section 5311f).

The first joint section 5311a has an approximately columnar shape, and supports, at a distal end (lower end) thereof, an upper end of the tubular part 5305 of the microscope unit 5303 such that the tubular part 5305 can rotate about a rotation axis (a first axis O₁) that is parallel to a central axis of the tubular part 5305. Here, the first joint section 5311a may be configured such that the first axis O₁ matches an optical axis of the imaging unit of the microscope unit 5303. With this configuration, by rotating the microscope unit 5303 about the first axis O₁, it is possible to change a visual field so as to rotate the captured image.

The first link 5313a fixedly supports the first joint section 5311a at a distal end thereof. Specifically, the first link 5313a is a bar-shaped member having an approximately L-shape, in which one side on a distal end side extends in a direction perpendicular to the first axis O₁ and an end portion of the one side is connected to the first joint section 5311a so as to come into contact with an upper end portion of an outer periphery of the first joint section 5311a. The second joint section 5311b is connected to an end portion of another side on a proximal end side of the approximately L-shape of the first link 5313a.

The second joint section 5311b has an approximately columnar shape, and supports, at a distal end thereof, the proximal end of the first link 5313a such that the first link 5313a can rotate about a rotation axis (a second axis O₂) that is perpendicular to the first axis O₁. A distal end of the second link 5313b is fixedly connected to a proximal end of the second joint section 5311b.

The second link 5313b is a bar-shaped member having an approximately L-shape, in which one side on a distal end side extends in a direction perpendicular to the second axis O₂ and an end portion of the one side is fixedly connected to the proximal end of the second joint section 5311b. The third joint section 5311c is connected to another side on a proximal end side of the approximately L-shape of the second link 5313b.

The third joint section 5311c has an approximately columnar shape, and supports, at a distal end thereof, a proximal end of the second link 5313b such that the second link 5313b can rotate about a rotation axis (a third axis O₃) that is perpendicular to both of the first axis O₁ and the second axis O₂. A distal end of the third link 5313c is fixedly connected to a proximal end of the third joint section 5311c. By rotating the components on the distal end side including the microscope unit 5303 about the second axis O₂ and the third axis O₃, it is possible to move the microscope unit 5303 such that the position of the microscope unit 5303 in a horizontal plane is changed. In other words, by controlling rotation about the second axis O₂ and the third axis O₃, it is possible to move the visual field of the captured image in a plane.

The third link 5313c is configured such that a distal end side has an approximately columnar shape, and the proximal end of the third joint section 5311c is fixedly connected to a distal end of the columnar shape such that both of the proximal end of the third joint section 5311c and the distal end of the columnar shape have approximately the same central axes. A proximal end side of the third link 5313c has a prismatic shape, and an end portion thereof is connected to the fourth joint section 5311d.

The fourth joint section 5311d has an approximately columnar shape, and supports, at a distal end thereof, the proximal end of the third link 5313c such that the third link 5313c can rotate about a rotation axis (a fourth axis O₄) that is perpendicular to the third axis O₃. A distal end of the fourth link 5313d is fixedly connected to a proximal end of the fourth joint section 5311d.

The fourth link 5313d is a bar-shaped member that extends in an approximately linear manner so as to be perpendicular to the fourth axis O₄, and is fixedly connected to the fourth joint section 5311d such that an end portion at a distal end thereof comes into contact with a side surface of the approximate columnar shape of the fourth joint section 5311d. The fifth joint section 5311e is connected to a proximal end of the fourth link 5313d.

The fifth joint section 5311e has an approximately columnar shape, and supports, at a distal end thereof, the proximal end of the fourth link 5313d such that the fourth link 5313d can rotate about a rotation axis (a fifth axis O₅) that is parallel to the fourth axis O₄. A distal end of the fifth link 5313e is fixedly connected to a proximal end of the fifth joint section 5311e. The fourth axis O₄ and the fifth axis O₅ are rotation axes along which the microscope unit 5303 can move in a vertical direction. By moving the components including the microscope unit 5303 about the fourth axis O₄ and the fifth axis O₅, it is possible to adjust a height of the microscope unit 5303, that is, a distance between the microscope unit 5303 and the observation target.

The fifth link 5313e is configured by combining a first member that has an approximately L-shape, in which one side extends in a vertical direction and another side extends in a horizontal direction, and a second member that has a bar shape that extends downward in the vertical direction from a certain portion of the first member that extends in the horizontal direction. The proximal end of the fifth joint section 5311e is fixedly connected to a certain portion in the vicinity of an upper end of a vertically extended portion of the first member of the fifth link 5313e. The sixth joint section 5311f is connected to a proximal end (lower end) of the second member of the fifth link 5313e.

The sixth joint section 5311f has an approximately columnar shape, and supports, at a distal end thereof, a proximal end of the fifth link 5313e such that the fifth link 5313e can rotate about a rotation axis (a sixth axis O₆) that is parallel to the vertical direction. A distal end of the sixth link 5313f is fixedly connected to a proximal end of the sixth joint section 5311f.

The sixth link 5313f is a bar-shaped member that extends in the vertical direction, and the proximal end thereof is fixedly connected to an upper surface of the base section 5315.

Rotatable ranges of the first joint section 5311a to the sixth joint section 5311f are appropriately set such that the microscope unit 5303 can move in a desired manner. With this configuration, in the arm section 5309 configured as described above, with respect to movement of the microscope unit 5303, 3 translation degrees of freedom and 3 rotational degrees of freedom, that is, a total of 6 degrees of freedom of movement, can be realized. In this manner, by configuring the arm section 5309 such that the 6 degrees of freedom can be realized with respect to the movement of the microscope unit 5303, it is possible to freely control the position and the posture of the microscope unit 5303 in the movable range of the arm section 5309. Therefore, it becomes possible to observe the surgical field from every angle, so that it is possible to more smoothly perform surgery.

Meanwhile, the configuration of the arm section 5309 illustrated in the drawing is one example, and the number and the shapes (lengths) of the links, the number of joint sections, arrangement positions, the directions of the rotation axes and the like in the arm section 5309 may be appropriately designed so as to realize a desired degree of freedom. For example, as described above, to freely move the microscope unit 5303, it is preferable to configure the arm section 5309 so as to realize the 6 degrees of freedom, but the arm section 5309 may be configured so as to have a larger degree of freedom (that is, redundant degree of freedom). If the redundant degree of freedom is present, in the arm section 5309, it is possible to change the posture of the arm section 5309 while the microscope unit 5303 is fixed. Therefore, for example, it is possible to realize control that is more convenient for the surgeon, such as controlling the posture of the arm section 5309 such that the arm section 5309 does not interfere with the visual field of the surgeon who is watching the display apparatus 50.

Here, the first joint section 5311a to the sixth joint section 5311f may include driving mechanisms, such as motors, and actuators including encoders or the like for detecting rotation angles in the respective joint sections. Further, by causing the control device 5317 to appropriately control driving of each of the actuators arranged in the first joint section 5311a to the sixth joint section 5311f, the posture of the arm section 5309, that is, the position and the posture of the microscope unit 5303 can be controlled. Specifically, the control device 5317 is able to recognize current posture of the arm section 5309 and a current position and current posture of the microscope unit 5303, on the basis of information on the rotation angles of the respective joint sections that are detected by the encoders. With use of the recognized information, the control device 5317 calculates a control value (for example, a rotation angle, generated torque, or the like) for each of the joint sections such that the microscope unit 5303 can move in a desired manner, and drives the driving mechanism of each of the joint sections in accordance with the control value. Meanwhile, in this case, a method of controlling the arm section 5309 by the control device 5317 is not limited, and various well-known control methods, such as force control or position control, may be adopted.

For example, it may be possible to allow the surgeon to appropriately perform operation input via an input device (not illustrated), cause the control device 5317 to appropriately control the arm section 5309 in accordance with the operation input, and control the position and the posture of the microscope unit 5303. Through the control as described above, it is possible to move the microscope unit 5303 from an arbitrary position to another arbitrary position, and thereafter fixedly support the microscope unit 5303 at the moved position. Meanwhile, it is preferable to adopt, as the input device, a device, such as a foot switch, that is operable even when the surgeon holds a surgical tool in his/her hand, in view of the convenience of the surgeon. Further, it may be possible to perform operation input in a non-contact manner, on the basis of gesture detection or a line-of-sight detection using a camera that is arranged in a wearable device or a surgery room. With this configuration, even a user in a clean zone can more freely operate a device that is located in a dirty zone. Alternatively, the arm section 5309 may be operated by what is called a master-slave system. In this case, the arm section 5309 may be remotely operated by a user via an input device that is installed in a place distant from the surgery room.

Furthermore, if fore control is adopted, what is called power assist control may be performed in which an external force is received from a user, and the actuators of the first joint section 5311a to the sixth joint section 5311f are driven such that arm section 5309 smoothly moves in accordance with the external force. With this configuration, when the user directly moves the the position of the microscope unit 5303 while holding the microscope unit 5303, it is possible to move the microscope unit 5303 with a relatively small force. Therefore, it is possible to more intuitively move the microscope unit 5303 by simpler operation, so that it is possible to improve convenience for the user.

Furthermore, driving of the arm section 5309 may be controlled such that the arm section 5309 performs pivot operation. Here, the pivot operation is operation of moving the microscope unit 5303 such that the optical axis of the microscope unit 5303 is always oriented toward a predetermined point (hereinafter, referred to as a pivot point) in a space. With the pivot operation, it is possible to observe the same observation position from various directions, so that it is possible to more precisely observe the affected area. Meanwhile, if the microscope unit 5303 is configured such that a focal length thereof is not adjustable, it is preferable to perform the pivot operation in a state in which a distance between the microscope unit 5303 and the pivot point is fixed. In this case, it is sufficient to adjust the distance between the microscope unit 5303 and the pivot point to a fixed focal length of the microscope unit 5303. With this configuration, the microscope unit 5303 moves on a hemispherical surface (schematically illustrated in FIG. 20) with a radius corresponding to the focal length centered at the pivot point, so that it is possible to obtain a clear captured image even if the observation direction is changed. In contrast, if the microscope unit 5303 is configured such that the focal length thereof is adjustable, it may be possible to perform the pivot operation in a state in which the distance between the microscope unit 5303 and the pivot point is changeable. In this case, for example, the control device 5317 may calculate the distance between the microscope unit 5303 and the pivot point on the basis of information on the rotation angle of each of the joint sections detected by the encoders, and automatically adjust the focal length of the microscope unit 5303 on the basis of a calculation result. Alternatively, if the microscope unit 5303 is provided with the AF function, it may be possible to automatically adjust the focal length by the AF function every time the distance between the microscope unit 5303 and the pivot point is changed due to the pivot operation.

The control device 5317 integrally controls operation of the microscope surgical system 5300 by controlling operation of the microscope device 5301 and the display apparatus 50. For example, the control device 5317 controls drive of the arm section 5309 by operating the actuators of the first joint section 5311a to the sixth joint section 5311f in accordance with a predetermined control method. Furthermore, for example, the control device 5317 changes the operation mode of the arm section 5309 by controlling operation of brakes of the first joint section 5311a to the sixth joint section 5311f. Moreover, the control device 5317 has the functions of the camera control unit 12a described in the first embodiment. Furthermore, the control device 5317 generates the surgical field image K(x, y), in which focus and exposure are adjusted to the region of interest, from the surgical field image K(x, y) that is captured by the imaging unit of the microscope unit 5303, and outputs the generated surgical field image K(x, y) to the display apparatus 50. Meanwhile, the control device 5317 may perform various kinds of well-known signal processing, such as a developing process (demosaicing process), a high-quality picture process (a bandwidth enhancement process, a super-resolution process, a noise reduction (NR) process, and/or a hand-shake correction process), on the surgical field image K(x, y) that is acquired by the microscope unit 5303 of the microscope device 5301.

Communication between the control device 5317 and the microscope unit 5303 and communication between the control device 5317 and the first joint section 5311a to the sixth joint section 5311f may be wired communication or wireless communication. In the case of the wired communication, the communication may be performed using electrical signals or using optical communication. In this case, transmission cables used for the wired communication may be configured as electrical signal cables, optical fibers, or composite cables of the electrical signal cables and the optical fibers. In contrast, in the case of the wireless communication, it is not necessary to install the transmission cables in the surgery room, so that it is possible to solve a problem in that movement of medical staff in the surgery room is interfered with by the transmission cables.

The control device 5317 may be a processor, such as a central processing unit (CPU) or a graphics processing unit (GPU), a microcomputer in which a processor and a storage element, such as a memory, are mounted, a control substrate, or the like. The processor of the control device 5317 operates in accordance with a predetermined program, so that various functions as described above can be implemented. Meanwhile, in the example illustrated in the drawings, the control device 5317 is arranged as a device separated from the microscope device 5301, but the control device 5317 may be arranged inside the base section 5315 of the microscope device 5301 and configured in an integrated manner with the microscope device 5301. Alternatively, the control device 5317 may be configured with a plurality of devices. For example, a microcomputer, a control substrate, and the like are arranged in each of the microscope unit 5303 and the first joint section 5311a to the sixth joint section 5311f of the arm section 5309, and they are connected in a communicable manner to thereby implement the same functions as those of the control device 5317.

The display apparatus 50 is arranged inside the surgery room and displays an image corresponding to image data that is generated by the control device 5317, under the control of the control device 5317. In other words, the display apparatus 50 displays the surgical field image K(x, y) that is captured by the microscope unit 5303. Meanwhile, the display apparatus 50 may display various kinds of information on surgery, such as body information on a patient or a procedure of surgery, instead of the surgical field image K(x, y) or in addition to the surgical field image K(x, y). In this case, display of the display apparatus 50 may be appropriately changed by operation performed by the user. Alternatively, it may be possible to arrange the plurality of display apparatuses 50, and display the surgical field image K(x, y) and various kinds of information on each of the display apparatuses 50. Meanwhile, as the display apparatus 50, various well-known display apparatuses, such as a liquid crystal display apparatus or an EL display apparatus, may be adopted.

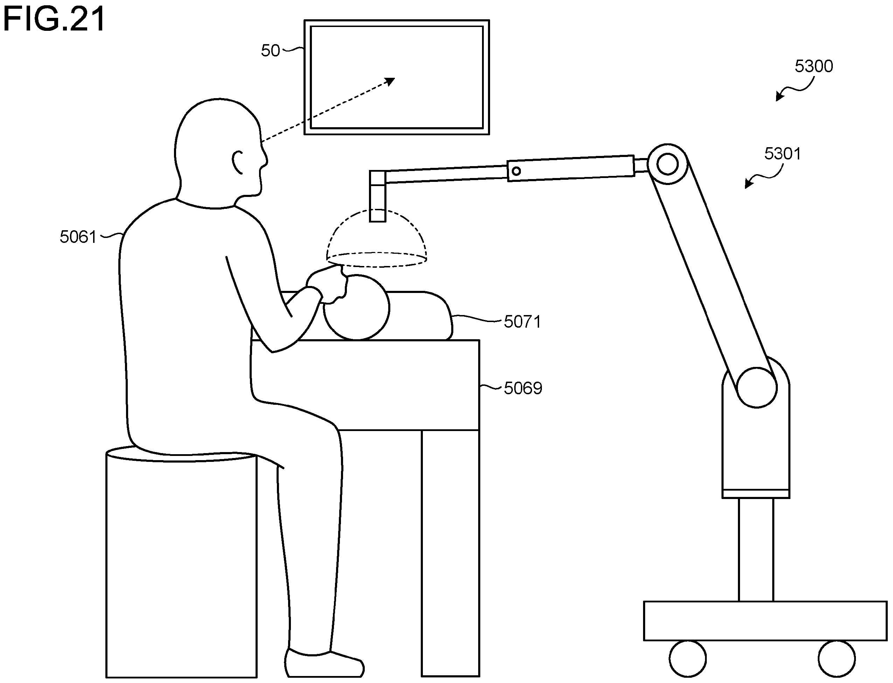

FIG. 21 is a diagram illustrating a situation in which a surgery is performed using the microscope surgical system 5300 illustrated in FIG. 20. In FIG. 21, a situation in which the surgeon 5061 uses the microscope surgical system 5300 and performs surgery for the patient 5071 on the patient bed 5069 is schematically illustrated. Meanwhile, in FIG. 21, for the sake of simplicity, the control device 5317 included in the microscope surgical system 5300 is not illustrated, and the microscope device 5301 including the microscope unit 5303 (FIG. 20) is illustrated in a simplified manner.

As illustrated in FIG. 21, at the time of surgery, the microscope surgical system 5300 displays the surgical field image K(x, y), which is captured by the microscope device 5301 using the imaging parameters adjusted by the control device 5317, on the display apparatus 50 that is installed on a wall surface of the surgery room. The display apparatus 50 is arranged at a position facing the surgeon 5061, and the surgeon 5061 performs various kinds of treatment, such as excision of the affected area, while observing the state of the surgical site using the surgical field image K(x, y), which is displayed on the display apparatus 50 and in which focus and exposure are adjusted to the surgical field.

As described above, according to the eleventh embodiment, the imaging unit is mounted on the microscope unit 5303. Therefore, when surgery is performed using a microscope, the microscope surgical system 5300 is able to output the surgical field image K(x, y) in which focus and exposure are continuously adjusted to the surgical field.

Thus, one example of the microscope surgical system 5300 to which the technique according to the present disclosure may be adopted has been described. Meanwhile, while the microscope surgical system 5300 has been described above as one example, a system to which the technique according to the present disclosure may be adopted is not limited to this example. For example, the microscope device 5301 may function as a support arm apparatus that supports, at a distal end thereof, a different observation apparatus or a different surgical tool, instead of the microscope unit 5303. As the different observation apparatus, for example, an endoscope may be adopted. Furthermore, as the different surgical tool, a forceps, tweezers, an insufflation tube for insufflation, an energy treatment tool that makes an incision in tissue or seals blood vessels by ablation, and the like may be adopted. By supporting the observation apparatus and the surgical tool as described above by the support arm apparatus, it is possible to more stably fix the position and reduce load on the medical staff as compared to a case in which the medial staff manually hold the observation apparatus and the surgical tool. The technique according to the present disclosure may be applied to the support arm apparatus that supports components other than the microscope unit as described above.

Meanwhile, the effects described in the present specification are mere example, and are not specifically limited, so that other effects may be achieved.

Furthermore, the embodiments of the present disclosure are not limited to the embodiments as described above, and various modifications may be made within the scope not departing from the gist of the present disclosure.

For example, as for the method of setting the region of interest as described above, the region-of-interest setting unit 20 may automatically set the region of interest instead of setting the detection frame by the user. In this case, the region-of-interest setting unit 20 sets, as the region of interest, a region that meets a certain condition in a screen on the basis of a trigger signal using a foot switch or the like by the user. For example, the region-of-interest setting unit 20 sets, as the region of interest, an object that meets any of a condition that an object occupies a predetermined area or larger in a screen at the time of acquisition of the trigger signal, a condition that an object is located on the frontmost side, and a condition that an object is located in the center, or an object that meets some of the conditions as described above. Furthermore, the region-of-interest setting unit 20 may include a classifier that performs learning in advance with machine learning algorithm (for example, machine learning algorithm using a multi-layer neural network) by using, as learning data, a plurality of surgical field images or the three-dimensional information in which the region of interest is set and that generates parameters (for example, a weight coefficient of each of layers of the multi-layer neural network), and set the region of interest based on the input surgical field image. Moreover, the trigger signal issued by the user may be generated based on press information on an arbitrary button of the camera head (for example, information on full press or half press).

Additionally, the present technology may also be configured as below.
(1) A medical observation system comprising:
   an imaging apparatus that obtains a surgical field image by capturing an image of a surgical field;
   a three-dimensional information generation unit that generates three-dimensional information on a surgical field from the surgical field image captured by the imaging apparatus;
   a setting unit that sets at least one region of interest in a surgical field image that is captured by the imaging apparatus at a predetermined timing;
   an estimation unit that estimates a relative position corresponding to the region of interest from a surgical field image that is captured at a certain timing different from the predetermined timing, on the basis of the three-dimensional information and information on the region of interest;
   an adjustment unit that adjusts a control parameter of the imaging apparatus when the imaging apparatus captures a surgical field image, on the basis of three-dimensional information and pixel values of a surgical field image corresponding to the relative position of the region of interest estimated by the estimation unit; and
   a display control unit that outputs a surgical field image that is captured by the imaging apparatus using the control parameter adjusted by the adjustment unit.
(2) The medical observation system according to (1), wherein
   the imaging apparatus includes a single imaging element, and
   the three-dimensional information generation unit generates the three-dimensional information on the surgical field on the basis of at least two surgical field images that are captured by the imaging apparatus at different times.
(3) The medical observation system according to (1), wherein
   the imaging apparatus includes two imaging elements that capture different ranges including portions overlapping with each other, and
   the three-dimensional information generation unit generates the three-dimensional information on the surgical field on the basis of two surgical field images that are captured by the imaging element at a same time.
(4) The medical observation system according to (1), wherein
   the imaging apparatus includes a distance measurement apparatus that measures a distance between a single imaging element and a target object, and
   the three-dimensional information generation unit generates the three-dimensional information on the surgical field on the basis of an image captured by the imaging element and a distance measured by the distance measurement apparatus.
(5) The medical observation system according to any one of (1) to (4), wherein the three-dimensional information generation unit generates three-dimensional information on a feature point that is extracted from one of an entire range or an arbitrary range of the surgical field image.
(6) The medical observation system according to any one of (1) to (5), wherein the control parameter is an optical parameter that defines a state of an optical system of the imaging apparatus.
(7) The medical observation system according to any one of (1) to (6), wherein the control parameter is an imaging parameter that defines an exposure condition of the imaging apparatus.
(8) The medical observation system according to any one of (1) to (7), wherein the control parameter is a development parameter that defines a development condition of the imaging apparatus.
(9) The medical observation system according to any one of (1) to (8), wherein the control parameter is a light emission parameter that defines a light emission state of a light source apparatus that applies illumination light to an imaging range of the imaging apparatus.
(10) The medical observation system according to (9), wherein
   the setting unit predicts a reflection direction of the illumination light emitted by the light source apparatus, on the basis of the three-dimensional information on the surgical field generated by the three-dimensional information generation unit, and
   when reflected light that travels in the predicted reflection direction is captured by the imaging apparatus, the setting unit eliminates a region in which the reflected light is captured from a target in which the region of interest is to be set.
(11) The medical observation system according to any one of (1) to (10), wherein the setting unit further includes a function to specify a distance range in which the region of interest is present, and sets a region of interest in the specified distance range.
(12) The medical observation system according to (5), further comprising:
   a detection unit that detects a registered object from the surgical field image, wherein
   the setting unit sets the feature point from a region except for the object detected by the detection unit in the surgical field image.
(13) The medical observation system according to any one of (1) to (12), wherein
   the adjustment unit changes an imaging magnification of a surgical field image captured by the imaging apparatus, in accordance with the estimated three-dimensional position of the region of interest, and
   the setting unit changes a size of the region of interest in accordance with the imaging magnification.
(14) The medical observation system according to any one of (1) to (13), wherein the adjustment unit sets an initial value of a control parameter when the imaging apparatus captures a surgical field image, in accordance with the estimated three-dimensional position of the region of interest.
(15) The medical observation system according to any one of (1) to (14), wherein the imaging apparatus is mounted on an endoscope.
(16) The medical observation system according to any one of (1) to (14), wherein the imaging apparatus is mounted on a microscope.
(17) A medical observation apparatus comprising:
   a three-dimensional information generation unit that generates three-dimensional information on a surgical field from a surgical field image that is obtained by capturing an image of a surgical field;
   a setting unit that sets at least one region of interest in a surgical field image captured at a predetermined timing;
   an estimation unit that estimates a relative position corresponding to the region of interest from a surgical field image that is captured at a certain timing different from the predetermined timing, on the basis of the three-dimensional information and information on the region of interest;
   an adjustment unit that adjusts a control parameter at the time of capturing a surgical field image, on the basis of three-dimensional information and pixel values of a surgical field image corresponding to the relative position of the region of interest estimated by the estimation unit; and
   a display control unit that outputs a surgical field image that is captured using the control parameter adjusted by the adjustment unit.
(18) A medical observation method including:
   a step of generating three-dimensional information on a surgical field from a surgical field image that is obtained by capturing an image of a surgical field;
   a step of setting at least one region of interest in a surgical field image that is captured at a predetermined timing;
   a step of estimating a relative position corresponding to the region of interest from a surgical field image that is captured at a certain timing different from the predetermined timing, on the basis of the three-dimensional information and a position of the region of interest;
   a step of adjusting a control parameter at the time of capturing a surgical field image, on the basis of three-dimensional information and pixel values of a surgical field image corresponding to the estimated relative position of the region of interest; and
   a step of displaying a surgical field image that is captured using the adjusted control parameter.

### Reference Signs List

10a, 10b, 10c, 10d, 10e, 10f medical observation system
12a, 12b, 12c camera control unit (medical observation apparatus)
14 three-dimensional information generation unit
15 map generation unit (detection unit)
16 subject position estimation unit
18 development processing unit
20 region-of-interest setting unit (setting unit)
22 detection region estimation unit (estimation unit)
24 three-dimensional map data storage unit
26 parameter control unit (adjustment unit)
40 display control unit
42a, 42b, 42c, 42d imaging apparatus
44a, 44b, 44c, 44d imaging element
46 imaging plane phase difference sensor
48 depth sensor (distance measurement apparatus)
50 display apparatus
110 frame of interest
5001 endoscope
5061 surgeon
5062 scopist
5063 assistant
5300 microscope surgical system
5303 microscope unit
5317 control device
D(X, Y, Z) three-dimensional map (three-dimensional information)
K(x, y), K(x, y, t) surgical field image
L (x, y) enlarged surgical field image

## Claims

1. A medical observation system comprising:
an imaging apparatus that obtains a surgical field image by capturing an image of a surgical field;
a three-dimensional information generation unit that generates three-dimensional information on a surgical field from the surgical field image captured by the imaging apparatus;
a setting unit that sets at least one region of interest in a surgical field image that is captured by the imaging apparatus at a predetermined timing;
an estimation unit that estimates a relative position corresponding to the region of interest from a surgical field image that is captured at a certain timing different from the predetermined timing, on the basis of the three-dimensional information and information on the region of interest;
an adjustment unit that adjusts a control parameter of the imaging apparatus when the imaging apparatus captures a surgical field image, on the basis of three-dimensional information and pixel values of a surgical field image corresponding to the relative position of the region of interest estimated by the estimation unit; and
a display control unit that outputs a surgical field image that is captured by the imaging apparatus using the control parameter adjusted by the adjustment unit.

2. The medical observation system according to claim 1, wherein
the imaging apparatus includes a single imaging element, and
the three-dimensional information generation unit generates the three-dimensional information on the surgical field on the basis of at least two surgical field images that are captured by the imaging apparatus at different times.

3. The medical observation system according to claim 1, wherein
the imaging apparatus includes two imaging elements that capture different ranges including portions overlapping with each other, and
the three-dimensional information generation unit generates the three-dimensional information on the surgical field on the basis of two surgical field images that are captured by the imaging element at a same time.

4. The medical observation system according to claim 1, wherein
the imaging apparatus includes a distance measurement apparatus that measures a distance between a single imaging element and a target object, and
the three-dimensional information generation unit generates the three-dimensional information on the surgical field on the basis of an image captured by the imaging element and a distance measured by the distance measurement apparatus.

5. The medical observation system according to claim 1, wherein the three-dimensional information generation unit generates three-dimensional information on a feature point that is extracted from one of an entire range or an arbitrary range of the surgical field image.

6. The medical observation system according to claim 1, wherein the control parameter is an optical parameter that defines a state of an optical system of the imaging apparatus.

7. The medical observation system according to claim 1, wherein the control parameter is an imaging parameter that defines an exposure condition of the imaging apparatus.

8. The medical observation system according to claim 1, wherein the control parameter is a development parameter that defines a development condition of the imaging apparatus.

9. The medical observation system according to claim 1, wherein the control parameter is a light emission parameter that defines a light emission state of a light source apparatus that applies illumination light to an imaging range of the imaging apparatus.

10. The medical observation system according to claim 9, wherein
the setting unit predicts a reflection direction of the illumination light emitted by the light source apparatus, on the basis of the three-dimensional information on the surgical field generated by the three-dimensional information generation unit, and
when reflected light that travels in the predicted reflection direction is captured by the imaging apparatus, the setting unit eliminates a region in which the reflected light is captured from a target in which the region of interest is to be set.

11. The medical observation system according to claim 1, wherein the setting unit further includes a function to specify a distance range in which the region of interest is present, and sets a region of interest in the specified distance range.

12. The medical observation system according to claim 5, further comprising:
a detection unit that detects a registered object from the surgical field image, wherein
the setting unit sets the feature point from a region except for the object detected by the detection unit in the surgical field image.

13. The medical observation system according to claim 1, wherein
the adjustment unit changes an imaging magnification of a surgical field image captured by the imaging apparatus, in accordance with the estimated three-dimensional position of the region of interest, and
the setting unit changes a size of the region of interest in accordance with the imaging magnification.

14. The medical observation system according to claim 1, wherein the adjustment unit sets an initial value of a control parameter when the imaging apparatus captures a surgical field image, in accordance with the estimated three-dimensional position of the region of interest.

15. The medical observation system according to claim 1, wherein the imaging apparatus is mounted on an endoscope.

16. The medical observation system according to claim 1, wherein the imaging apparatus is mounted on a microscope.

17. A medical observation apparatus comprising:
a three-dimensional information generation unit that generates three-dimensional information on a surgical field from a surgical field image that is obtained by capturing an image of a surgical field;
a setting unit that sets at least one region of interest in a surgical field image captured at a predetermined timing;
an estimation unit that estimates a relative position corresponding to the region of interest from a surgical field image that is captured at a certain timing different from the predetermined timing, on the basis of the three-dimensional information and information on the region of interest;
an adjustment unit that adjusts a control parameter at the time of capturing a surgical field image, on the basis of three-dimensional information and pixel values of a surgical field image corresponding to the relative position of the region of interest estimated by the estimation unit; and
a display control unit that outputs a surgical field image that is captured using the control parameter adjusted by the adjustment unit.

18. A medical observation method comprising:
a step of generating three-dimensional information on a surgical field from a surgical field image that is obtained by capturing an image of a surgical field;
a step of setting at least one region of interest in a surgical field image that is captured at a predetermined timing;
a step of estimating a relative position corresponding to the region of interest from a surgical field image that is captured at a certain timing different from the predetermined timing, on the basis of the three-dimensional information and a position of the region of interest;
a step of adjusting a control parameter at the time of capturing a surgical field image, on the basis of three-dimensional information and pixel values of a surgical field image corresponding to the estimated relative position of the region of interest; and
a step of outputting a surgical field image that is captured using the adjusted control parameter.
